(19) Europäisches Patentamt / European Patent Office / Office européen des brevets

(11) **EP 2 671 569 B1**

(12) **EUROPEAN PATENT SPECIFICATION**

(45) Date of publication and mention
of the grant of the patent:
**16.09.2015 Bulletin 2015/38**

(51) Int Cl.:
***A61K 9/16*** *(2006.01)*    ***A61K 9/20*** *(2006.01)*
***A61K 9/28*** *(2006.01)*

(21) Application number: **12004322.9**

(22) Date of filing: **06.06.2012**

(54) **Stable pharmaceutical compositions with fast onset**

Stabile pharmazeutische Zusammensetzungen mit schnell einsetzender Wirkung

Compositions pharmaceutiques stables avec début rapide

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.12.2013 Bulletin 2013/50**

(73) Proprietor: **Galenicum Health S.L.**
**08005 Barcelona (ES)**

(72) Inventor: **Gonzalez, Joaquim, Domingo**
**08005 Barcelona (ES)**

(74) Representative: **Galenicum Health S.L.**
**Avenida Diagonal 123, Floor 11**
**08005 Barcelona (ES)**

(56) References cited:
**EP-A1- 1 739 072**

• BLANCO M ET AL: "Near infrared spectroscopy in the study of polymorphic transformations", ANALYTICA CHIMICA ACTA, ELSEVIER, AMSTERDAM, NL, vol. 567, no. 2, 17 May 2006 (2006-05-17), pages 262-268, XP027912638, ISSN: 0003-2670 [retrieved on 2006-05-17]
• Bauer, Kurt H.: "Lehrbuch der pharmazeutischen Technologie", 1 January 1997 (1997-01-01), Georg Thieme Verlag, Stuttgart ISBN: 3-8047-1700-4 pages 313-316,
• Voigt, R.: "Pharmazeutische Technologie", 1 January 2006 (2006-01-01), Deutscher Apotheker Verlag, Stuttgart ISBN: 3-7692-3511-8 pages 272-276,
• Voigt, R.: "Pharmazeutische Technologie", 1 January 2006 (2006-01-01), Deutscher Apotheker Verlag, Stuttgart ISBN: 3-7692-3511-8 pages 254-255,

## Description

The present invention relates to processes to obtain pharmaceutical compositions comprising dexketoprofen trometamol and to pharmaceutical compositions obtained by said processes.

Throughout this application various publications, published patent applications, and patents are referenced. The disclosures of these documents in their entirety are hereby incorporated by reference into this application in order to more fully describe the state of the art to which this invention pertains.

## STATE OF THE ART

Dexketoprofen belongs to a class of medicines called non-steroidal antiinflammatory drugs (NSAIDs). It works by blocking the action of a substance in the body called cyclo-oxygenase. Cyclo-oxygenase is involved in the production of chemicals in the body called prostaglandins. Prostaglandins are produced in response to injury or certain diseases and would otherwise go on to cause swelling, inflammation and pain. By blocking cyclo-oxygenase, dexketoprofen prevents the production of prostaglandins and therefore reduces inflammation and pain. Along with Peripheral analgesic action it possesses central analgesic action.

Dexketoprofen was first disclosed in the WO8904658 The WO9411332 relates to a specific salt of dexketoprofen, concretely dexketoprofen trometamol salt. However, both WO8904658 and WO9411332 are also silent of how to specifically formulate the eitherdexketoprofen or dexketoprofen trometamol.

The EP1739072 relates to crystalline forms of dexketoprofen trometamol. The EP1739072 mentions that the dissolution profile of a tablet depends on the crystalline form of the dexketoprofen trometamol. During the prosecution of the EP1739072 the applicant mentioned that tablets obtained by direct compression dissolve faster when they comprise dexketoprofen trometamol in the so-called crystalline form A compared to tablets comprising comprising the so-called crystalline form B. Moreover, it is taught that wet granulation techniques should be avoided in case of dexketoprofen trometamol, due to the stability issues.

The dissolution profile is essential for the medical use of a drug. For example, a fast dissolution, to have the faster onset possible, is sometimes required for pharmaceutical compositions comprising agents for the treatment of pain. The dissolution profile of a drug does not only depend on the drug but also on the formulation and on the manufacturing process used to obtain it. According to Aulton, dissolution profiles are expected to be faster from tablets prepared by direct compression when compare to tablets prepared by granulation techniques (see M.E. Aulton, Pharmacuetics. The Science of Dosage Form Design. Page 404. Second Edition. 2002. Churchill Livingstone). For example, tablets comprising naproxen sodium made from the granules, prepared by wet granulation method using water, showed a significant decrease in solution as compared to those made by dry blending method (see Drug Development and Industrial Pharmacy, 1994, Vol. 20, No.13, Pages 2151-2156).

## SUMMARY OF THE INVENTION

Therefore, an object underlying the present invention is to provide a stable solid pharmaceutical composition comprising dexketoprofen trometamol which a fast onset. In contrast to the pharmaceutical compositions according to the prior art, the compositions as herein disclosed are not limited to a specific crystalline form in order to have a fast onset. The compositions as herein disclosed have a faster bioavailability, compare to the ones obtained by simple mixture, even when the composition obtained by direct compression and the composition as herein disclosed has the same dissolution profile in vitro. The pharmaceutical composition according to the present invention offers the advantage of a low content of total impurities originating from the decomposition of dexketoprofen trometamol. The manufacturing processes as herein disclosed produce granules with excellent properties to be compressed to form tablets.

The granulates as herein disclosed and the powders as herein disclosed, i.e. the mixture formed with granulate and the extra-granular ingredients, have a good flowability. Good flow characteristics are necessary because the mechanical action of the tablet press requires a volume of fill. A tablet press does not weigh the precise amount of powder for each tablet. To achieve consistent tablet weights, the formula must be designed to flow consistently and to fill volumetrically. Thus, the granulates as herein disclosed and the powders as herein disclosed possess a consistent particle-size distribution and density to attain proper flow and achieve volume of fill (i.e., tablet weight). In other words, the granulates as herein disclosed and the powders as herein disclosed flow consistently and consistent results are attained. The granulates as herein disclosed and the powders as herein disclosed fit into the die cavity (the place where powders are filled on the tablet press) and they compress correctly. The tablets as herein disclosed do not fall apart, the active ingredient is in all the tablets (content uniformity).

If dexketoprofen trometamol represents a very small portion of the overall tablet, then one of the challenge is to ensure that each tablet has the same amount of active ingredient. dexketoprofen trometamol segregates in some circumstances from the other ingredients in the blending process. The ingredients may be incompatible because of particle size, particle density, flow characteristics, compressibility, and water content. These incompatibilities cause problems such as segregation during blending or during transfer of the product to the press as well as separation of the active on the tablet press. The processes as herein disclosed are one solution to the segregation problem. In addition, the pharmaceutical compositions as herein disclosed contains the correct amount of active ingredient, even if the active is only a small percentage of the tablet ingredients.

The granulates as herein disclosed and the powders as herein disclosed flow, compress, eject from the tablet press and disintegrate properly, even when dexketoprofen trometamol represents a high overall percentage of the total tablet. Even in this case, dexketoprofen trometamol do not always cooperate. The nature of the active must be understood and its characteristics may have to be improved to make this process work. Some actives are very fine, small particles that are lighter than other particles. Even if the active is the correct size it may not flow smoothly, and flowability is very important when making a good tablet. Furthermore, the active could be the right particle size and it may flow well, but it may not blend well with the other ingredients. The active ingredient may be too dry or too moist, which prevents proper compression. The compositions as herein disclosed solve this problem, by granulating the dexketoprofen trometamol, blended with the other ingredients in the formulation, and compressed on the tablet press as herein disclosed. The manufacturing process as herein disclosed produced good results even when different dexketoprofen trometamol are used (e.g. particle size, water content). Another advantage of the processes and compositions as herein disclosed is that small tablets which are easy to swallow are provided.

In the first aspect, the invention provides a process for the manufacture of an oral pharmaceutical composition comprising dexketoprofen trometamol in the form of tablets or granules, wherein the pharmaceutical composition comprises between 3 % and 60 % by weight of dexketoprofen trometamol in respect of the total amount of the pharmaceutical composition, wherein the total amount of lubricant or lubricants present in the pharmaceutical composition ranges from 0.5 to 6 % by weight in respect of the total amount of the pharmaceutical composition; wherein the total amount of disintegrant or disintegrants present in the pharmaceutical composition ranges from 2 to 20 % by weight in respect of the total amount of the pharmaceutical composition; wherein the total amount of diluent or diluents present in the pharmaceutical composition ranges from 15 % to 85 % by weight in respect of the total amount of the pharmaceutical composition, wherein the process for the manufacture of the pharmaceutical composition comprises the steps of:

i) sieving when appropriate the intra-granular ingredients to form uniformly sized particles of intra-granular ingredients;

ii) either mixing the particles of intra-granular ingredients and dexketoprofen trometamol, preferably in a granulator-mixer, granulate the obtained mixture with at least one binder, preferably the binder comprises water, more preferably consists essentially of water; even more preferably consists of water; wherein the final water content of the granulate is from about 5 % to about 30 % by weight;

iii) or mixing the particles of intra-granular ingredients, preferably in a granulator-mixer; granulate the obtained mixture with a solution or dispersion of dexketoprofen trometamol in water; wherein the final water content of the granulate is from about 5 % to about 30 % by weight;

iv) optionally and/or if required, performing a sieving of the granulate obtained in either step (ii) or step (iii):

v) drying the granulate of the previous step (ii), (iii) or (iv) to an endpoint water content of less than about 8 % by weight, preferably less than 6.5 by weight, more preferably from about 1.0 % to 5.5 % by weight, wherein the drying is preferably carried out in a fluid bed at an input temperature between 35 and 105 °C;

vi) mixing the granulate of the previous step with the extra-granular agents and blending to form a blend;

vii) and, when the pharmaceutical composition is in the form of tablets, compressing the blend into tablets using a tablet press.

The processes as herein disclosed are preferably conducted in a closed system. The closed system may be a fluid bed, a granulator or a coater equipment Specifically, once the initial ingredients are added to the system (intra-granular tableting ingredients, dexketoprofen trometamol, a binder and extra-granular tableting agents), their exposure to the atmosphere is reduced as much as practicable. Accordingly, the system is designed to reduce exposure of the ingredients to atmospheric conditions by connecting the components thereof such that minimal exposure to the atmosphere outside the system is achieved. This is done to reduce the chance of exposure to excess atmospheric moisture and light, for example, which could adversely affect the desired stability properties of the end product The extragranular ingredients can also be sieved as in step (i) for the intra-granular ingredients. The drying step can be carried out in one or several.

In the second aspect, the present invention provides a pharmaceutical composition comprising the active ingredient as defined in the first aspect.

In the third aspect, the present invention provides a granulate comprising between 3 % and 60 % by weight of dexketoprofen trometamol in respect of the total amount of the granule, at least one disintegrant and at least one diluent.

In the fourth aspect, the present invention provides a process for the manufacture of a granulate comprising dexketoprofen trometamol, wherein the process comprises the following steps:

i) sieving when appropriate the intra-granular ingredients to form uniformly sized particles of intra-granular ingredients;

ii) either mixing the particles of intra-granular ingredients and dexketoprofen trometamol, preferably in a granulator-mixer; granulate the obtained mixture with at least one binder, preferably the binder comprises water, more preferably consists essentially of water, even more preferably consists of water; wherein the final water content of the granulate is from about 5 % to about 30 % by weight;

iii) or mixing the particles of intra-granular ingredients, preferably in a granulator-mixer; granulate the obtained mixture with a solution or dispersion of dexketoprofen trometamol in water, wherein the final water content of the granulate is from about 5 % to about 30 % by weight;

iv) optionally and/or if required, performing a sieving of the granulate obtained in either step (ii) or step (iii);

v) drying the granulate of the previous step (ii), (iii) or (iv) to an endpoint water content of less than about 8 % by weight, preferably less than 6.5 by weight, more preferably from about 1.0 % to 5.5 % by weight, wherein the drying is preferably carried out in a fluid bed at an input temperature between 35 and 105 °C.

In the fifth aspect, the present invention provides a granulate obtained by the process of fourth aspect.

In the sixth aspect, the present invention provides a pharmaceutical composition comprising the granulate as defined in the previous aspects.

In the sixth aspect, the present invention provides the use of the granulate as defined in the previous aspect for the manufacture of a tablet

## DEFINITIONS

When used herein, the term "sieving" refers to a separation process which is based on the difference in the size of particles of a material. In a particular embodiment, the process uses a mechanical sieve or screen. For example, a sieve can be in the form of a regular or irregular mesh, a perforated solid surface, a three-dimensional matrix, or a column of differential porosity. The term encompasses a process separating a larger or coarser particle from a smaller or finer particle. In an embodiment, separation is achieved with the assistance of equipment using vibratory motion or sifters.

The term "intragranular component" refers to the ingredients of the compressed tablet that are incorporated prior to the granulation step, and "extra-granular component" refers to the ingredients that are incorporated after granulation.

The term "diluent" as used herein, refers to an agent or mixture of agents that when added to a formulation, make that formulation thinner or less concentrated and may also improve manufacturability. Diluents can be used to stabilize compounds because they can provide a more stable environment. In certain embodiments, diluents increase the bulk of the composition to facilitate compression or create sufficient bulk for a homogenous blend for capsule filling. Such compounds include e.g., lactose, starch, mannitol, sorbitol, dextrose, cellulose, microcrystalline cellulose such as Avicel ®, dibasic calcium phosphate, dicalcium phosphate dihydrate, tricalcium phosphate, calcium phosphate, lactose, anhydrous lactose, spray-dried lactose; pregelatinized starch, compressible sugar, such as Di-Pac ® (Amstar), hydroxypropylmethylcellulose, hydroxypropylmethylcellulose acetate, sucrose-based diluents, confectioner's suga,; monobasic calcium sulfate monohydrate, calcium sulfate dihydrate, calcium lactate trihydrate, dextrates, hydrolyzed cereal solids, amylosem, powdered cellulose, calcium carbonate, glycine, kaolin, sodium chloride; inositol, bentonite, and the like.

The terms "binder" or "binding agent" refer to any substance or mixture that exerts a physicochemical attractive force between molecules, and hence may be used in the formulation of a dosage form. In one embodiment of the invention, the binder or the binding agent may be mixed with other components of the composition, so that it is distributed uniformly throughout the dosage form. In one embodiment the binding agent is formed by the dissolution or dispersion of a binder in a liquid to form a binding solution or dispersion, and preferably the liquid is water. In another embodiment the binder is water. The binder may also provide a matrix upon which any additional components can associate. Binders include, but are not limited to, gelatin, polyvinylpyrrolidone (PVP), hydroxypropyl methylcellulose (HPMC), starch grades (pregelatinized or plain), hydroxypropylcellulose (HPC), and carboxymethylcellulose (CMC).

As used herein, the term "granulation" refers to the process in which primary powder particles are made to adhere to form larger, multiparticle entities called granules. Pharmaceutical granules typically have an average size range between 0.2 and 4.0 mm, depending on their subsequent use. In the majority of cases this will be in the production of tablets or capsules, when granules will be made as an intermediate product and have a typical average size range between 0.2 and 0.5 mm, but larger granules are used as a dosage form in their own right. Granulation normally commences after initial dry mixing of the necessary powdered ingredients so that a uniform distribution of each ingredient through the mix is achieved. After granulation the granules will either be packed (when used as a dosage form), or they may be mixed with other excipients prior to tablet compaction or capsule filling.

As used herein, the term "wet granulation" refers to the process of adding a liquid solution to powders, is one of the most common ways to granulate. It involves the massing of a mix of dry primary powder particles using a granulating fluid. The fluid contains a solvent which must be volatile so that it can be removed by drying, and be non-toxic. Typical liquids include water, ethanol and isopropanol either alone or in combination. The liquid solution can be either aqueous based or solvent based. Aqueous solutions have the advantage of being safer to deal with than solvents. The granulation liquid may be used alone or, more usually, as a solvent containing a dissolved adhesive (also referred to as a binder or binding agent) which is used to ensure particle adhesion once the granule is dry.

As used herein, "lubricant" means a substance that reduces friction between the composition of the present invention and the surfaces of the apparatus used to compact the composition into a compressed form. Suitable lubricants for the present invention include, for example, fatty acids, such as palmitic acid, stearic acid, oleic acid, hydrogenated vegetable oils, triglycerides of fatty acids, metal salts of fatty acids, such as for example, zinc stearate and magnesium stearate, glycols, such as polyethylene glycol, and talc, as well as mixtures thereof. Others well known lubricants are sodium benzoate, adipic acid, fumaric acid, glyceryl monostearate, glycerol distearate, gyceryl behenate, glyceryl palmitostearate, glycerol distearate. In one embodiment, the lubricant component of the composition of the present invention comprises magnesium stearate and glycerol distearate.

As used herein, "disintegrant" means a substance or a mixture of substances added to a tablet to facilitate its breakup or disintegration after administration. Materials serving as disintegrants used to be classified chemically as starches, clays, celluloses, alginates, gums, and cross-linked polymers. Other miscellaneous disintegrants include surfactants, natural sponge, resins, effervescent mixtures, and hydrous aluminum silicate. Suitable disintegrants include one or more of water-soluble disintegrants, such as pregelatinized starch, sodium carboxymethyl cellulose and sodium alginate, and water- insoluble cross-linked polymers, such as cross-linked carboxymethylcellulose, croscarmellose sodium also known as crosslinked sodium carboxymethylcellulose, sodium starch glycolate and cross-linked povidone also known as crosslinked polyvinylpyrrolidone. These water-insoluble cross-linked polymers, known as super disintegrants, are completely effective when used at low relative amounts (e.g. 2 to 4% by weight of the tablet). It is postulated that the rate, force, and extent of swelling play an important role in these disintegrants that work by swelling.

As used herein, the term "stable" refers to a solid pharmaceutical composition comprising dexketoprofen trometamol wherein the total content of impurities originating from the decomposition of dexketoprofen trometamol does not exceed 10 % area, preferably 6 % area, more preferably 4 % area and most preferably 2 % area determined by liquid chromatography at 220 nm if such a composition is stored for 7 days at 60°C in a closed vessel. Therefore, another aspect of the present invention relates to a stable solid pharmaceutical composition comprising dexketoprofen trometamol wherein the total content of impurities originating from the decomposition of dexketoprofen trometamol does not exceed 10 % area, preferably 6 % area, more preferably 4 % area and most preferably 2 % area determined by liquid chromatography if such a composition is stored for 7 days at 60 °C in a closed vessel.

According to the European Pharmacopoeia 7.0, 2.2.33, the loss on drying is measured as follows:

Loss on drying is the loss of mass expressed as per cent m/m.
Method. Place the prescribed quantity of the substance to be examined in a weighing bottle previously dried under the conditions prescribed for the substance to be examined. Dry the substance to constant mass or for the prescribed time by one of the following procedures. Where the drying temperature is indicated by a single value rather than a range, drying is carried out at the prescribed temperature $\pm$ 2 °C.

a) "in a desiccator" : the drying is carried out over diphosphorus pentoxide R at atmospheric pressure and at room temperature;
b) "in vacuo" : the drying is carried out over diphosphorus pentoxide R, at a pressure of 1.5 kPa to 2.5 kPa at room temperature;
c) "in vacuo within a specified temperature range" : the drying is carried out over diphosphorus pentoxide R, at a pressure of 1.5 kPa to 2.5 kPa within the temperature range prescribed in the monograph ;

d) "in an oven within a specified temperature range" : the drying is carried out in an oven within the temperature range prescribed in the monograph;
e) "under high vacuum": the drying is carried out over diphosphorus pentoxide R at a pressure not exceeding 0.1 kPa, at the temperature prescribed in the monograph.

In the summary of the invention and this description, each numerical value should be read once as modified by the term "about" (unless already expressly so modified), and then read again as not so modified unless otherwise indicated in context. All percents, parts, and ratios herein are by weight unless specifically noted otherwise.

**DETAILED DESCRIPTION OF THE INVENTION**

The process as herein disclosed for the manufacture of an oral pharmaceutical composition comprising dexketoprofen trometamol in the form of tablets or granules, wherein the pharmaceutical composition comprises between 3 % and 60 % by weight of dexketoprofen trometamol in respect of the total amount of the pharmaceutical composition, wherein the total amount of lubricant or lubricants present in the pharmaceutical composition ranges from 0.5 to 6 % by weight in respect of the total amount of the pharmaceutical composition; wherein the total amount of disintegrant or disintegrants present in the pharmaceutical composition ranges from 2 to 20 % by weight in respect of the total amount of the pharmaceutical composition; wherein the total amount of diluent or diluents present in the pharmaceutical composition ranges from 15 % to 85 % by weight in respect of the total amount of the pharmaceutical composition, wherein the process for the manufacture of the pharmaceutical composition comprises the steps of:

i) sieving when appropriate the intra-granular ingredients to form uniformly sized particles of intra-granular ingredients;

ii) either mixing the particles of intra-granular ingredients and dexketoprofen trometamol, preferably in a granulator-mixer; granulate the obtained mixture with at least one binder, preferably the binder comprises water, more preferably consists essentially of water, even more preferably consists of water; wherein the final water content of the granulate is from about 5 % to about 30 % by weight;

iii) or mixing the particles of intra-granular ingredients, preferably in a granulator-mixer; granulate the obtained mixture with a solution or dispersion of dexketoprofen trometamol in water; wherein the final water content of the granulate is from about 5 % to about 30 % by weight;

iv) optionally and/or if required, performing a sieving of the granulate obtained in either step (ii) or step (iii);

v) drying the granulate of the previous step (ii), (iii) or (iv) to an endpoint water content of less than about 8 % by weight, preferably less than 6.5 by weight, more preferably from about 1.0 % to 5.5 % by weight, wherein the drying is preferably carried out in a fluid bed at an input temperature between 35 and 105 °C;

vi) mixing the granulate of the previous step with the extra-granular agents and blending to form a blend;

vii) and, when the pharmaceutical composition is in the form of tablets, compressing the blend into tablets using a tablet press.

In a preferred embodiment the binder used in the processes as herein disclosed comprises water. Preferably, the binder consists essentially of water; even more preferably consists of water.
In a preferred embodiment, the pharmaceutical composition comprises between a 5 % and a 45 % by weight of dexketoprofen trometamol in respect of the total amount of the pharmaceutical composition. Preferably, the pharmaceutical composition comprises between a 7 % and a 30 % by weight of dexketoprofen trometamol in respect of the total amount of the pharmaceutical composition. More preferably, the pharmaceutical composition comprises between a 10 % and a 20 % by weight of dexketoprofen trometamol in respect of the total amount of the pharmaceutical composition.
The total amount of lubricant or lubricants present in the pharmaceutical composition can range from 1 to 5 % by weight, preferably from 1.2 to 3.5 % by weight, more preferably from 1.5 to 2.6 % by weight. The lubricants used in the pharmaceutical compositions as herein disclosed or granulates as herein disclosed can be selected from fatty acids, such as palmitic acid, stearic acid, oleic acid. hydrogenated vegetable oils, triglycerides of fatty acids, metal salts of fatty acids, such as for example, zinc stearate and magnesium stearate, glycols, such as polyethylene glycol, and talc, sodium benzoate, adipic acid, fumaric acid, glyceryl monostearate, glycerol distearate, gyceryl behenate, glyceryl palmitostearate as well as mixtures thereof. Preferred lubricants are magnesium stearate, glycerol distearate, stearic acid or mixtures thereof, more preferably the pharmaceutical composition comprises glycerol distearate.

The total amount of disintegrant or disintegrants present in the pharmaceutical composition can range from 4 to 16 % by weight, preferably from 8 to 14 % by weight The disintegrants used in the pharmaceutical compositions as herein disclosed or in the granulate as herein disclosed can be selected from starches, clays, celluloses, alginates, gums, and cross-linked polymers, surfactants, natural sponge, resins, effervescent mixtures, and hydrous aluminum silicate, water-soluble disintegrant, such as pregelatinized starch, sodium carboxymethyl cellulose and sodium alginate, and water-insoluble cross-linked polymers, such as cross-linked carboxymethylcellulose, croscarmellose sodium also known as crosslinked sodium carboxymethylcellulose, sodium starch glycolate and cross-linked povidone also known as crosslinked polyvinylpyrrolidone. Preferred disintegrants are selected from water-soluble disintegrants, such as pregelatinized starch, sodium carboxymethyl cellulose and sodium alginate, more preferably the disintegrant is sodium carboxymethyl cellulose.

The total amount of diluent or diluents present in the pharmaceutical composition ranges preferably from 25 % to 75 % by weight, more preferably from 35 % to 65 % by weight, even more preferably from 45 % 60 % by weight Useful diluents for the pharmaceutical compositions as herein disclosed and the granulate as herein disclosed are selected from lactose, starch, mannitol, sorbitol, dextrose, cellulose, microcrystalline cellulose, dibasic calcium phosphate, dicalcium phosphate dihydrate, tricalcium phosphate, calcium phosphate; lactose, anhydrous lactose, spray-dried lactose, pregelatinized starch, compressible sugar, hydroxypropylmethylcellulose, hydroxypropylmethylcellulose acetate, monobasic calcium sulfate monohydrate, calcium sulfate dihydrate, calcium lactate trihydrate, dextrates; hydrolyzed cereal solids, amylosem, powdered cellulose, calcium carbonate; glycine and any mixture thereof. Preferred diluents are lactose, calcium phosphate and microcrystalline cellulose, more preferably the diluent microcrystalline cellulose.

As to the solvent used in the wet granulation, it has been found the dexketoprofen trometamol is compatible with water during the granulation process when dexketoprofen trometamol is granulated according to the to process as herein disclosed. The liquid solution can be either aqueous based or solvent based. Surprisingly, the compositions as herein disclosed are stable, even when they are manufactured by aqueous wet granulation techniques. Aqueous solutions have many advantages, as for example the advantage of being safer to deal with than solvents. In the process as herein disclosed, water mixed with the intragranular ingredients form bonds between powder particles that are strong enough to lock them together. However, once the water dries, the powders forming the granulate obtained by the process as herein disclosed do not fall apart. Therefore, water is strong enough to create and hold a bond in the pharmaceutical compositions as herein disclosed. In most of the cases of the processes as herein disclosed water suffices to create the bonds, and a liquid solution that includes a binder is not always required. In a preferred embodiment, the final water content of the granulate of step (ii) or (iii) is from 12 % to 25 % by weight. More preferably, the final water content of the granulate of step (ii) or (iii) is from 14 % to 21 % by weight.

The drying process must be monitored. If the granulate is not enough dried, the water entrapped within the drug affects the stability of the final composition. In contrast, if granules have been too much dried, the granules will stick to the tablet-press tooling. Moreover, the water content in step {v} has an effect in the in the hardness and sticking problems of the tablets. In a preferred embodiment of the process as herein disclosed, in step (v) of the process as herein disclosed the granulate of the previous step (ii), (iii) or (iv) is dried to an endpoint water content from 1.0 % to 5.5 % by weight. In a more preferred embodiment, in step (v) the granulate of the previous step (ii), (iii) or (iv) is dried to an endpoint water content from 1.5% to 4.5 % by weight. The water content is measured by the method of the European Pharmacopoeia 7.0 2.2.32 (Loss on drying) page 51. The loss on drying is determined in a oven at 100°C for 15 minutes using 5 g of sample. More preferably, in step (v) the granulate of the previous step (ii), (iii) or (iv) is dried to an endpoint water content from 1.6 % to 3.5 % by weight The drying of the granulate normally is carried out in a fluid bed at an input temperature between 35 and 105 °C, preferably at an input temperature between 40 and 80 °C, more preferably at an input temperature between 45 and 65°C, even most preferably at an input temperature between 47 and 57 °C. In one of the most preferred embodiments, the drying of the granulate in step (v) is carried out in a fluid bed at an input temperature about 50 °C.

In a preferred embodiment of the process as herein disclosed, the granulate is blended in step (vi) with at least one excipient selected from a lubricant, a diluent, a disintegrant or any mixture thereof.

The pharmaceutical compositions as herein disclosed are preferably in the form of tablets, more preferably in the form of coated tablets.

Optionally, cores/tablets can be coated with conventional materials used for film coating, i.e. as described in Pharmaceutical Coating Technology (G. Cole (ed.), 1995). Film coating formulations usually contain the following components:

- polymer(s),
- plasticizer(s),
- colourant(s)/opacifier(s),
- vehicle (s).

In the film coating suspension minor quantities of flavours, surfactants and waxes can be used.

The majority of the polymers used in film coating are either cellulose derivatives, such as the cellulose ethers, or acrylic polymers and copolymers. Occasionally encountered are high molecular weight polyethylene glycols, such as for example macrogols, polyvinylpyrrolidone, polyvinylalcohol and waxy materials. Their function usually is to prevent bad mouth feel and/or taste and in some cases degradation, e.g. oxidation of the active ingredients and/or excipients used.

Typical cellulose ethers which may be applied as coatings are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The compositions as herein disclosed are more preferably an immediate release dosage form. An immediate release dosage form has to be understood as a dosage form having a dissolution performance such that about 80 % or more of the dexketoprofen trometamol or salt thereof contained in said pharmaceutical composition dissolves within 60 minutes. In a preferred embodiment, the immediately release composition as herein disclosed releases at least 80 % of dexketoprofen trometamol when placed in a paddle apparatus in 900 mL of buffered aqueous media at 37 $\pm$ 0,5 °C at 75 $\pm$ 1 revolutions per minute for 60 minutes under the following pH conditions: buffered aqueous media at a pH of 6.8 at 37 °C at 75 revolutions per minute for 60 minutes. In another embodiment of the pharmaceutical composition releases at least a 80 % in 45 min, preferably in 35 min and more preferably in 30 min in the aforementioned conditions. In another embodiment of the pharmaceutical composition releases at least a 80 % in 15 min, and at least a 95 % in 60 min in the aforementioned conditions. In another embodiment of the pharmaceutical composition releases at least a 80 % in 30 min, and at least a 95 % in 60 min in the aforementioned conditions. The phosphate buffer is prepared by mixing 750 mL of a solution HCL 1 N (8.3 mL/L of 37 % w/v) and 250 mL of a solution of $Na_3PO_4 \cdot H_2O$ 0.2 M (26.23 mg/mL). Adjust the pH to 6.8 with HCl 2N (8.3 mL/50 mL of HCl 37%) or with NaOH 2N (8 g/100mL).

In an embodiment, the dexketoprofen trometamol used in step (ii) or the dexketoprofen trometamol used to form the solution or dispersion of step (iii) is in a crystalline, amorphous form or mixtures thereof. In one embodiment, the dexketoprofen trometamol used to form the solution or dispersion of step (iii) is in a crystalline form. In another embodiment, the dexketoprofen trometamol used in step (ii) is in a crystalline form.

In a preferred embodiment, the dexketoprofen trometamol used in step (ii) is in a crystalline form A, B, C or mixtures thereof. In preferred embodiment, the dexketoprofen trometamol used in step (ii) is dexketoprofen trometamol crystalline form A. In preferred embodiment, the dexketoprofen trometamol used in step (ii) is dexketoprofen trometamol crystalline form B. In preferred embodiment, the dexketoprofen trometamol used in step (ii) is dexketoprofen trometamol crystalline form C. dexketoprofen trometamol crystalline form A and B are as disclosed in the EP1739072. dexketoprofen trometamol crystalline form C is the crystalline form obtained in the WO94/11332 example 1. In view of the results obtained, it is expected that form A and form C are in fact the same crystalline form.

The dexketoprofen trometamol used in step (ii) or the dexketoprofen trometamol used to form the solution or dispersion of step (iii) has typically an average particle size from 1 to 250 microns as measured by laser light diffraction. Preferably, the dexketoprofen trometamol used in step (ii) or the dexketoprofen trometamol used to form the solution or dispersion of step (iii) has an average particle size from 5 to 90 microns as measured by laser light diffraction. The use of laser light diffraction to measure particle sizes is a widely known technique. Laser diffraction is a particle size method which uses the average relative angular intensity of scattered light. Instruments that uses laser light diffraction to measure particle size have been available for many years from different manufactures (see e.g. US 5,610,712). The use of a small mean particle size leads to poor flowability and segregation problems of the mixture during the compression of the pharmaceutical compositions as herein disclosed. In turn, the use of active ingredient particles with a great mean particle size increases the drug release time and reduces the reproducibility of the release profile.

In one embodiment of the pharmaceutical compositions as herein disclosed the pharmaceutical composition comprises less than 5%, preferably less than 3, more preferably less than 1% of the L-ketoprofen in respect of the total amount of dexketoprofen trometamol.

In one embodiment, the pharmaceutical compositions herein disclosed comprises 16 and 20 mg of dexketoprofen trometamol by unit dosis. In another embodiment, the pharmaceutical compositions herein disclosed comprises 33 and 40 mg of dexketoprofen trometamol by unit dosis.

In yet another embodiment of the pharmaceutical composition as herein disclosed, the pharmaceutical composition, preferably a tablet, has a weight of less than 750 mg, and preferably comprises between 33 and 40 mg of dexketoprofen trometamol by unit dosis. Preferably the pharmaceutical composition has a weight of less than 500 mg. More preferably the pharmaceutical composition has a weight of less than 350 mg. Even more preferably, the pharmaceutical composition has a weight of about 260 mg.

In yet another embodiment of the pharmaceutical composition as herein disclosed, the pharmaceutical composition, preferably a tablet, has a weight of less than 750 mg, and preferably comprises between 16 and 20 mg of dexketoprofen trometamol by unit dosis. Preferably the pharmaceutical composition has a weight of less than 500 mg.

More preferably, the pharmaceutical composition has a weight of less than 350 mg. Even more preferably, the pharmaceutical composition has a weight of about 130 mg.

In another embodiment, it is provided an oral pharmaceutical composition comprising dexketoprofen trometamol and manufactured by wet granulation techniques. Preferably, the oral pharmaceutical compositions is manufactured by the processes as herein disclosed.

It is also provided a granulate comprising between 3 % and 60 % by weight of dexketoprofen trometamol in respect of the total amount of the granule, at least one disintegrant and at least one diluent. Preferably, the granulate comprises between a 5 % and a 45 % by weight of dexketoprofen trometamol in respect of the total amount of the granulate. More preferably, the granulate comprises between a 10 % and a 45 % by weight of dexketoprofen trometamol in respect of the total amount of the granulate. Even more preferably, the granulate comprises between a 16 % and a 30 % by weight of dexketoprofen trometamol in respect of the total amount of the granulate.

In another embodiment, the total amount of disintegrant or disintegrants present in the granulate ranges from 0.1 to 15 % by weight, preferably ranges from 0.2 to 10 % by weight, more preferably ranges from 0.5 to 4 % by weight.

In another embodiment, the total amount of diluent or diluents present in the granulate ranges from 15 % to 85 % by weight, preferably ranges from 25 to 75 % by weight, more preferably ranges from 35 to 65 % by weight, even more preferably ranges from 40 to 60 % by weight.

In another embodiment, the total amount water content is less than 8 % w/w, preferably the total amount water content is less than 6.5 % w/w, more preferably the water content is from 1.0 % to 5.5 % by weight, even more preferably the total amount water content is from 1.5% to 4.5 % by weight, even yet more preferably the total amount water content is from 1.6 % to 3.5 % by weight.

The granulate can be manufacture by the same process steps as the process of the first aspect of the invention. The process for the manufacturing the granulate comprises the following steps:

i) sieving when appropriate the intra-granular ingredients to form uniformly sized particles of intra-granular ingredients;

ii) either mixing the particles of intra-granular ingredients and dexketoprofen trometamol, preferably in a granulator-mixer; granulate the obtained mixture with at least one binder, preferably the binder comprises water, more preferably consists essentially of water; even more preferably consists of water, wherein the final water content of the granulate is from about 5 % to about 30 % by weight,

iii) or mixing the particles of intra-granular ingredients, preferably in a granulator-mixer, granulate the obtained mixture with a solution or dispersion of dexketoprofen trometamol in water, wherein the final water content of the granulate is from about 5 % to about 30 % by weight;

iv) optionally and/or if required, performing a sieving of the granulate obtained in either step (ii) or step (iii);

v) drying the granulate of the previous step (ii), (iii) or (iv) to an endpoint water content of less than about 8 % by weight, preferably less than 6.5 by weight, more preferably from about 1.0 % to 5.5 % by weight, wherein the drying is preferably carried out in a fluid bed at an input temperature between 35 and 105 °C.

In another embodiment, it is provided a granulate obtained by the above process.

The pharmaceutical composition as herein disclosed are useful as medicament, concretely for the treatment of pain. In all the aspects and embodiments, the present invention provides a pharmaceutical composition useful for the symptomatic treatment of pain of mild to moderate intensity, such a musculo-skeletal pain, dysmenorrhoea or dental pain.

Further aspect and embodiments of the present invention can be found in the following numbered clauses:

Clause 1.- A process for the manufacture of an oral pharmaceutical composition comprising dexketoprofen trometamol in the form of tablets or granules, wherein the pharmaceutical composition comprises between 3 % and 60 % by weight of dexketoprofen trometamol in respect of the total amount of the pharmaceutical composition, wherein the total amount of lubricant or lubricants present in the pharmaceutical composition ranges from 0.5 to 6 % by weight in respect of the total amount of the pharmaceutical composition; wherein the total amount of disintegrant or disintegrants present in the pharmaceutical composition ranges from 2 to 20 % by weight in respect of the total amount of the pharmaceutical composition; wherein the total amount of diluent or diluents present in the pharmaceutical composition ranges from 15 % to 85 % by weight in respect of the total amount of the pharmaceutical composition, wherein the process for the manufacture of the pharmaceutical composition comprises the steps of:

i) sieving when appropriate the intra-granular ingredients to form uniformly sized particles of intra-granular ingredients;

ii) either mixing the particles of intra-granular ingredients and dexketoprofen trometamol, preferably in a granulator-mixer, granulate the obtained mixture with at least one binder, preferably the binder comprises water, more preferably consists essentially of water; even more preferably consists of water; wherein the final water content of the granulate is from about 5 % to about 30 % by weight;

iii) or mixing the particles of intra-granular ingredients, preferably in a granulator-mixer; granulate the obtained mixture with a solution or dispersion of dexketoprofen trometamol in water; wherein the final water content of the granulate is from about 5 % to about 30 % by weight;

iv) optionally and/or if required, performing a sieving of the granulate obtained in either step (ii) or step (iii);

v) drying the granulate of the previous step (ii), (iii) or (iv) to an endpoint water content of less than about 8 % by weight, preferably less than 6.5 by weight, more preferably from about 1.0 % to 5.5 % by weight, wherein the drying is preferably carried out in a fluid bed at an input temperature between 35 and 105 °C;

vi) mixing the granulate of the previous step with the extra-granular agents and blending to form a blend;

vii) and, when the pharmaceutical composition is in the form of tablets, compressing the blend into tablets using a tablet press.

Clause 2.- The process according to the preceding clause, wherein the binder comprises water. One possibly is that the binding solution is in water.

Clause 3.- The process according to any one of the preceding clauses, wherein the binder consists essentially of water; even more preferably consists of water.

Clause 4.- The process according to any one of the preceding clauses, wherein the binder consists of water.

Clause 5.- The process according to any one of the preceding clauses, wherein the pharmaceutical composition comprises between a 5 % and a 45 % by weight of dexketoprofen trometamol in respect of the total amount of the pharmaceutical composition.

Clause 6.- The process according to any one of the preceding clauses, wherein the pharmaceutical composition comprises between a 7 % and a 30 % by weight of dexketoprofen trometamol in respect of the total amount of the pharmaceutical composition.

Clause 7.- The process according to any one of the preceding clauses, wherein the pharmaceutical composition comprises between a 10 % and a 20 % by weight of dexketoprofen trometamol in respect of the total amount of the pharmaceutical composition.

Clause 8.- The process according to any one of the preceding clauses, wherein the total amount of lubricant or lubricants present in the pharmaceutical composition ranges from 1 to 5 % by weight.

Clause 9.- The process according to any one of the preceding clauses, wherein the total amount of lubricant or lubricants present in the pharmaceutical composition ranges from 1.2 to 3.5 % by weight.

Clause 10.- The process according to any one of the preceding clauses, wherein the total amount of lubricant or lubricants present in the pharmaceutical composition ranges from 1.5 to 2.6 % by weight

Clause 11.- The process according to any one of the preceding clauses, wherein the pharmaceutical comprises at least a lubricant selected from fatty acids, such as palmitic acid, stearic acid, oleic acid, hydrogenated vegetable oils, triglycerides of fatty acids, metal salts of fatty acids, such as for example, zinc stearate and magnesium stearate, glycols, such as polyethylene glycol, and talc, sodium benzoate, adipic acid, fumaric acid, glyceryl monostearate, glycerol distearate, gyceryl behenate, glyceryl palmitostearate as well as mixtures thereof.

Clause 12.- The process according to any one of the preceding clauses, wherein the pharmaceutical comprises magnesium stearate, glycerol distearate, stearic acid or mixtures thereof, more preferably the pharmaceutical composition comprises glycerol distearate.

Clause 13.- The process according to any one of the preceding clauses, wherein the total amount of disintegrant or disintegrants present in the pharmaceutical composition ranges from 4 to 16 % by weight.

Clause 14.- The process according to any one of the preceding clauses, wherein the total amount of disintegrant or disintegrants present in the pharmaceutical composition ranges from 8 to 14 % by weight.

Clause 15.- The process according to any one of the preceding clauses, wherein the pharmaceutical comprises at least a disintegrant selected from starches, clays, celluloses, alginates, gums, and cross-linked polymers, surfactants, natural sponge, resins, effervescent mixtures, and hydrous aluminum silicate, water-soluble disintegrant, such as pregelatinized starch, sodium carboxymethyl cellulose and sodium alginate, and water-insoluble cross-linked polymers, such as cross-linked carboxymethylcellulose, croscarmellose sodium also known as cross-linked sodium carboxymethylcellulose, sodium starch glycolate and cross-linked povidone also known as crosslinked polyvinylpyrrolidone.

Clause 16.- The process according to any one of the preceding clauses, wherein the pharmaceutical comprises at least a disintegrant selected from water-soluble disintegrants, such as pregelatinized starch, sodium carboxymethyl cellulose and sodium alginate, more preferably the pharmaceutical composition comprises sodium carboxymethyl cellulose.

Clause 17.- The process according to any one of the preceding clauses, wherein the total amount of diluent or diluents present in the pharmaceutical composition ranges from 25 % to 75 % by weight.

Clause 18.- The process according to any one of the preceding clauses, wherein the total amount of diluent or diluents present in the pharmaceutical composition ranges from 35 % to 65 % by weight

Clause 19.- The process according to any one of the preceding clauses, wherein the total amount of diluent or diluents present in the pharmaceutical composition ranges from 45 % 60 % by weight.

Clause 20.- The process according to any one of the preceding clauses, wherein the pharmaceutical comprises at least a diluent selected from lactose, starch, mannitol, sorbitol, dextrose, cellulose, microcrystalline cellulose, dibasic calcium phosphate, dicalcium phosphate dihydrate, tricalcium phosphate, calcium phosphate; lactose, anhydrous lactose, spray-dried lactose, pregelatinized starch, compressible sugar, hydroxypropylmethylcellulose, hydroxypropylmethylcellulose acetate, monobasic calcium sulfate monohydrate, calcium sulfate dihydrate, calcium lactate trihydrate, dextrates: hydrolyzed cereal solids, amylosem, powdered cellulose, calcium carbonate; glycine and any mixture thereof.

Clause 21.- The process according to any one of the preceding clauses, wherein the pharmaceutical comprises at least a diluent, such as lactose, calcium phosphate and microcrystalline cellulose, more preferably the pharmaceutical composition comprises microcrystalline cellulose.

Clause 22.- The process according to any one of the preceding clauses, wherein the final water content of the granulate of step (ii) or (iii) is from 12 % to 25 % by weight.

Clause 23.- The process according to any one of the preceding clauses, wherein the final water content of the granulate of step (ii) or (iii) is from 14 % to 21 % by weight.

Clause 24.- The process according to any one of the preceding clauses, wherein in step (v) the granulate of the previous step (ii), (iii) or (iv) is dried to an endpoint water content from 1.0 % to 5.5 % by weight

Clause 25.- The process according to any one of the preceding clauses, wherein in step (v) the granulate of the previous step (ii), (iii) or (iv) is dried to an endpoint water content from 1.5% to 4.5 % by weight

Clause 26.- The process according to any one of the preceding clauses, wherein in step (v) the granulate of the previous step (ii), (iii) or (iv) is dried to an endpoint water content from 1.6 % to 3.5 % by weight

Clause 27.- The process according to any one of the preceding clauses, wherein in step (v) the drying of the granulate is carried out in a fluid bed at an input temperature between 35 and 105 °C.

Clause 28.- The process according to any one of the preceding clauses, wherein in step (v) the drying of the granulate is carried out in a fluid bed at an input temperature between 40 and 80 °C.

Clause 29.- The process according to any one of the preceding clauses, wherein in step (v) the drying of the granulate is carried out in a fluid bed at an input temperature between 45 and 65 °C.

Clause 30.- The process according to any one of the preceding clauses, wherein in step (v) the drying of the granulate is carried out in a fluid bed at an input temperature between 47 and 57 °C.

Clause 31.- The process according to any one of the preceding clauses, wherein in step (v) the drying of the granulate is carried out in a fluid bed at an input temperature about 50 °C.

Clause 32.- The process according to any one of the preceding clauses, wherein in step (vi) the granulate is blended with at least one excipient selected from a lubricant, a diluent, a disintegrant or any mixture thereof.

Clause 33.- The process according to any one of the preceding clauses, wherein the pharmaceutical composition is in the form of tablets.

Clause 34.- The process according to any one of the preceding clauses, wherein the pharmaceutical composition is in the form of coated tablets.

Clause 35.- The process according to any one of the preceding clauses, wherein the dexketoprofen trometamol used in step (ii) or the dexketoprofen trometamol used to form the solution or dispersion of step (iii) is in a crystalline, amorphous form or mixtures thereof.

Clause 36.- The process according to the preceding clause, wherein the dexketoprofen trometamol used in step (ii) or the dexketoprofen trometamol used to form the solution or dispersion of step (iii) is in a crystalline form.

Clause 37.- The process according to the preceding clause, wherein the dexketoprofen trometamol used in step (ii) is in a crystalline form.

Clause 38.- The process according to the preceding clause, wherein the dexketoprofen trometamol used in step (ii) is in a crystalline form A, B, C or mixtures thereof.

Clause 39.- The process according to any one of the three preceding clauses, wherein the dexketoprofen trometamol used in step (ii) is dexketoprofen trometamol crystalline form A.

Clause 40.- The process according to any one of the clauses 35 to 38, wherein the dexketoprofen trometamol used in step (ii) is dexketoprofen trometamol crystalline form B.

Clause 41.- The process according to any one of the clauses 35 to 38, wherein the dexketoprofen trometamol used in step (ii) is dexketoprofen trometamol crystalline form C.

Clause 42.- The process according to any one of the preceding clauses, wherein the dexketoprofen trometamol used in step (ii) or the dexketoprofen trometamol used to form the solution or dispersion of step (iii) has an average particle size from 1 to 250 microns as measured by laser light diffraction.

Clause 43.- The process according to any one of the preceding clauses, wherein the dexketoprofen trometamol used in step (ii) or the dexketoprofen trometamol used to form the solution or dispersion of step (iii) has an average particle size from 5 to 90 microns as measured by laser light diffraction.

Clause 44.- The process according to any one of the preceding clauses, wherein the pharmaceutical composition comprises between 33 and 40 mg of dexketoprofen trometamol by unit dosis.

Clause 45.- The process according to any one of the preceding clauses, wherein the pharmaceutical composition comprises between 16 and 20 mg of dexketoprofen trometamol by unit dosis.

Clause 46.- The oral pharmaceutical composition in the form of tablets or granules comprising the active ingredient as defined in any one of the clauses 1 to 45.

Clause 47.- The pharmaceutical composition according to the preceding clause, wherein the pharmaceutical compositions is manufactured by wet granulation techniques.

Clause 48.- The pharmaceutical composition according to any one of the two preceding clauses manufactured by any one of the processes as defined in any one of the preceding process clauses.

Clause 49.- The pharmaceutical composition according to any one of the preceding three clauses for the symptomatic treatment of pain of mild to moderate intensity, such a musculo-skeletal pain, dysmenorrhoea or dental pain.

Clause 50.- A granulate comprising between 3 % and 60 % by weight of dexketoprofen trometamol in respect of the total amount of the granule, at least one disintegrant and at least one diluent.

Clause 51.- The granulate according to the preceding clause, wherein the granulate comprises between a 5 % and a 45 % by weight of dexketoprofen trometamol in respect of the total amount of the granulate.

Clause 52.- The granulate according to any one of the two preceding clauses, wherein the granulate comprises between a 10 % and a 45 % by weight of dexketoprofen trometamol in respect of the total amount of the granulate.

Clause 53.- The granulate according to any one of the three preceding clauses, wherein the granulate comprises between a 16 % and a 30 % by weight of dexketoprofen trometamol in respect of the total amount of the granulate.

Clause 54.- The granulate according to any one of the four preceding clauses, wherein the total amount of disintegrant or disintegrants present in the granulate ranges from 0.1 to 15 % by weight.

Clause 55.- The granulate according to any one of the five preceding clauses, wherein the total amount of disintegrant or disintegrants present in the granulate ranges from 0.2 to 10 % by weight

Clause 56.- The granulate according to any one of the six preceding clauses, wherein the total amount of disintegrant or disintegrants present in the granulate ranges from 0.5 to 4 % by weight.

Clause 57.- The granulate according to any one of the seven preceding clauses, wherein the total amount of diluent or diluents present in the granulate ranges from 15 % to 85 % by weight

Clause 68.- The granulate according to any one of the eight preceding clauses. wherein the total amount of diluent or diluents present in the granulate ranges from 25 to 75% by weight.

Clause 59.- The granulate according to any one of the nine preceding clauses, wherein the total amount of diluent or diluents present in the granulate ranges from 35 to 65 % by weight.

Clause 60.- The granulate according to any one of the ten preceding clauses, wherein the total amount of diluent or diluents present in the granulate ranges from 40 to 60 % by weight.

Clause 61.- The granulate according to any one of the eleven preceding clauses, wherein the total amount water content is less than 8 % w/w.

Clause 62.- The granulate according to any one of the twelve preceding clauses, wherein the total amount water content is less than 6.5 % w/w.

Clause 63.- The granulate according to any one of the thirteen preceding clauses, wherein the total amount water content is from 1.0 % to 5.5 % by weight.

Clause 64.- The granulate according to any one of the fourteen preceding clauses, wherein the total amount water content is from 1.5% to 4.5 % by weight

Clause 65.- The granulate according to any one of the fifteen preceding clauses, wherein the total amount water content is from 1.6 % to 3.5 % by weight.

Clause 66.- A process for the manufacture of the granulate as defined in clauses 50 to 65, wherein the process comprises the following steps:

i) sieving when appropriate the intra-granular ingredients to form uniformly sized particles of intra-granular ingredients;

ii) either mixing the particles of intra-granular ingredients and dexketoprofen trometamol, preferably in a granulator-mixer; granulate the obtained mixture with at least one binder, preferably the binder comprises water, more preferably consists essentially of water; even more preferably consists of water; wherein the final water content of the granulate is from about 5 % to about 30 % by weight;

iii) or mixing the particles of intra-granular ingredients, preferably in a granulator-mixer; granulate the obtained mixture with a solution or dispersion of dexketoprofen trometamol in water; wherein the final water content of the granulate is from about 5 % to about 30 % by weight;

iv) optionally and/or if required, performing a sieving of the granulate obtained in either step (ii) or step (iii);

v) drying the granulate of the previous step (ii), (iii) or (iv) to an endpoint water content of less than about 8 % by weight, preferably less than 6.5 by weight, more preferably from about 1.0 % to 5.5 % by weight, wherein the drying is preferably carried out in a fluid bed at an input temperature between 35 and 105 °C.

Clause 67.- The granulate obtained by the process of the preceding clause.

Clause 68.- A pharmaceutical composition comprising the granulate as defined in any one of the clauses 50 to 65, or 67.

Clause 69 - A pharmaceutical composition according to any one of the clauses 46 to 49 comprising the granulates according to clauses 50 to 65, or 67.

Clause 70.- The pharmaceutical composition according to any one of the two preceding clauses for the symptomatic treatment of pain of mild to moderate intensity, such a musculo-skeletal pain, dysmenorrhoea or dental pain.

Clause 71.- A powder comprising the granulate as defined in any one of the clauses 50 to 65, or 67.

Clause 72.- Use of a granulate according to any of the clauses 50 to 65, or 76 or the powder of clause 71 for the manufacture of a tablet, preferably coated.

Preferred combinations of the above clauses are as follows:

| Clause 73 | 1, 2, 36 and 42. |
| --- | --- |
| Clause 74 | 1, 3, 38 and 42. |
| Clause 75 | 1, 2, 5, 8, and 36. |
| Clause 76 | 1, 2, 5, 8, and 38. |
| Clause 77 | 1, 2, 39 and 42. |
| Clause 78 | 1, 2, 40 and 42. |
| Clause 79 | 1, 2, 41 and 42. |
| Clause 80 | 1, 2, 5, 8, 11, 13 and 15. |
| Clause 81 | 1, 2, 5, 8, 11, 13, 15 and 24. |
| Clause 82 | 1, 2, 5, 8, 11, 13, 15 and 25. |
| Clause 83 | 1, 2, 5, 8, 11, 13, 15 and 17. |

(continued)

| | |
|---|---|
| Clause 84 | 1, 2, 5, 8, 11, 13, 15 and 20. |
| Clause 85 | 1, 2, 5, 8, 11, 13, 15, 17 and 20. |
| Clause 86 | 1, 2, 22, 36 and 42. |
| Clause 87 | 1, 2, 24, 36 and 42. |
| Clause 88 | 1, 2, 5, 8, 22, and 36. |
| Clause 89 | 1, 2, 5, 8, 24 and 36. |
| Clause 90 | 1, 2, 5, 8, 25 and 36. |
| Clause 91 | 1, 2, 5, 8, 11, 13, 15, 22 and 24. |
| Clause 92 | 1, 2, 5, 8, 11, 13, 15, 22 and 25. |
| Clause 93 | 1, 2, 6, 8, 13, 17, 22, 24, 27, 32, 33, 38, 42 and 44. |
| Clause 94 | 1, 2, 6, 8, 13, 18, 22, 24, 27, 32, 33, 38, 42 and 44. |
| Clause 95 | 1, 2, 6, 8, 13, 17, 22, 24, 27, 32, 33, 41, 42 and 44. |
| Clause 98 | 1, 2, 6, 8, 13, 17, 22, 24, 27, 32, 33, 41, 43 and 44. |
| Clause 97 | 1, 8, 11 and 13 |
| Clause 98 | 1,17, 20 and 24 |
| Clause 99 | 1, 26, 29,39 and 42 |
| Clause 100 | 1, 24, 28, 39, 43, and 44 |
| Clause 101 | 1, 2, 6, 12, 14, 16, 18, 19, 21, 23, 25, 27, 33, 36, 39, 4 and 44. |
| Clause 102 | 1, 2, 6, 12, 14, 16, 18, 19, 21, 23, 25, 27, 33, 36, 39, 4 and 45. |
| Clause 103 | 1, 3, 6, 12, 14, 16, 18, 19, 21, 23, 25, 27, 33, 36, 39, 4 and 44. |
| Clause 104 | 50, 54 and 57 |
| Clause 105 | 104 and 61 |
| Clause 106 | 104 and 62 |
| Clause 107 | 104 and 63 |
| Clause 108 | 50, 52, 54, and 57 |
| Clause 109 | the composition of clause 48 and (any process according to clauses 73 to 103) |
| Clause 110 | the composition of clause 48 and the granulate according to any clauses 104 to 107 |

This invention will be better understood from the experimental details which follow. However, one skilled in the art will readily appreciate that the specific methods and results discussed are merely illustrative of the invention as described more fully in the claims which follow thereafter.

DESCRIPTION OF THE FIGURES

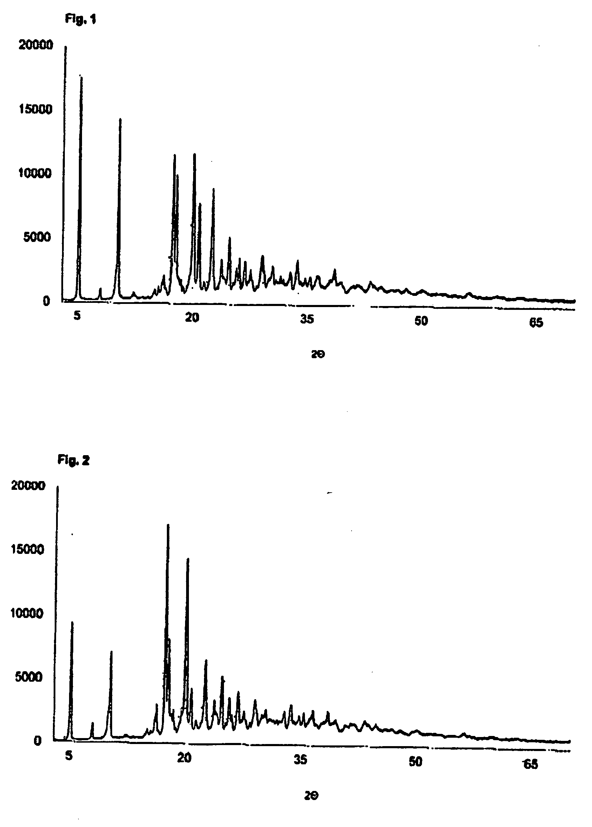

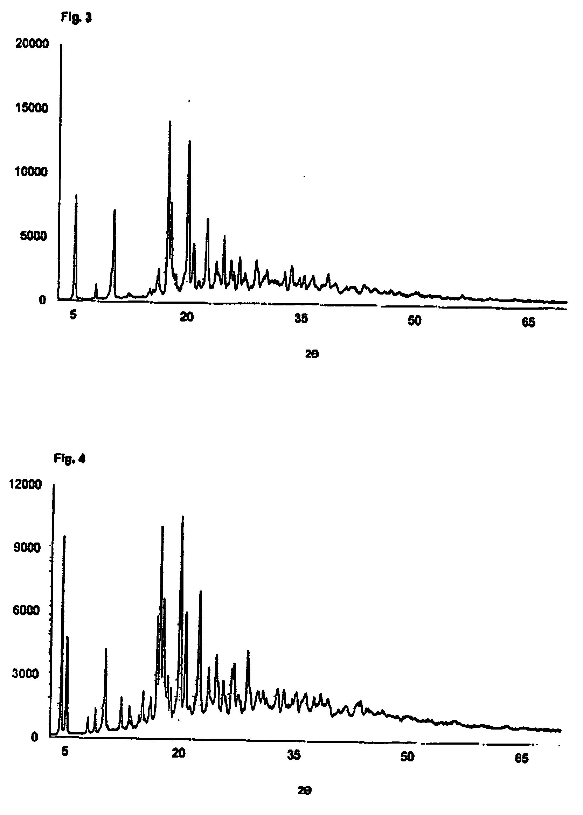

Fig. 1 to 4 depict the XRD of some of crystalline forms of dexketoprofen trometamol used in the process as herein disclosed. X and Y axis correspond to the values of 20 (Theta) (deg) and intensity (in units of intensity), respectively.

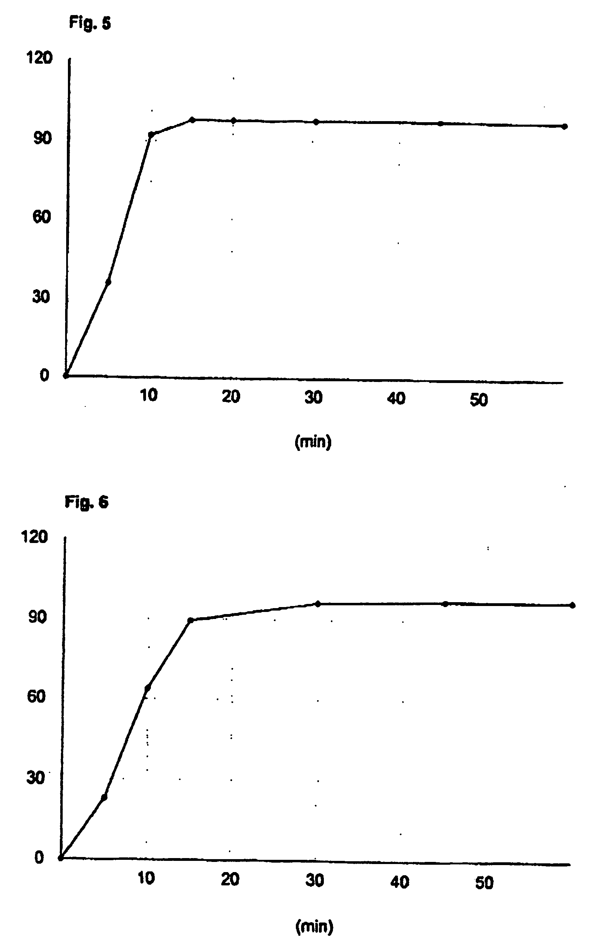

Fig. 5 and 6 correspond to dexketoprofen trometamol 12.5 mg dissolution profile (Ex. 1) for pH 6.8 and 1.2, respectively.

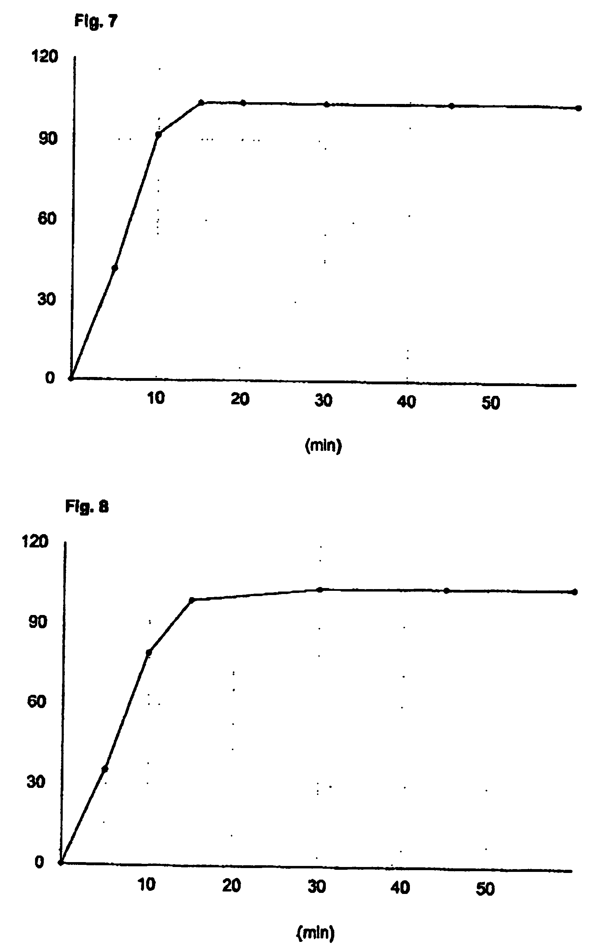

Fig. 7 and 8 correspond to dexketoprofen trometamol 12.5 mg dissolution profile (Ex. 2) for pH 6.8 and 1.2, respectively.

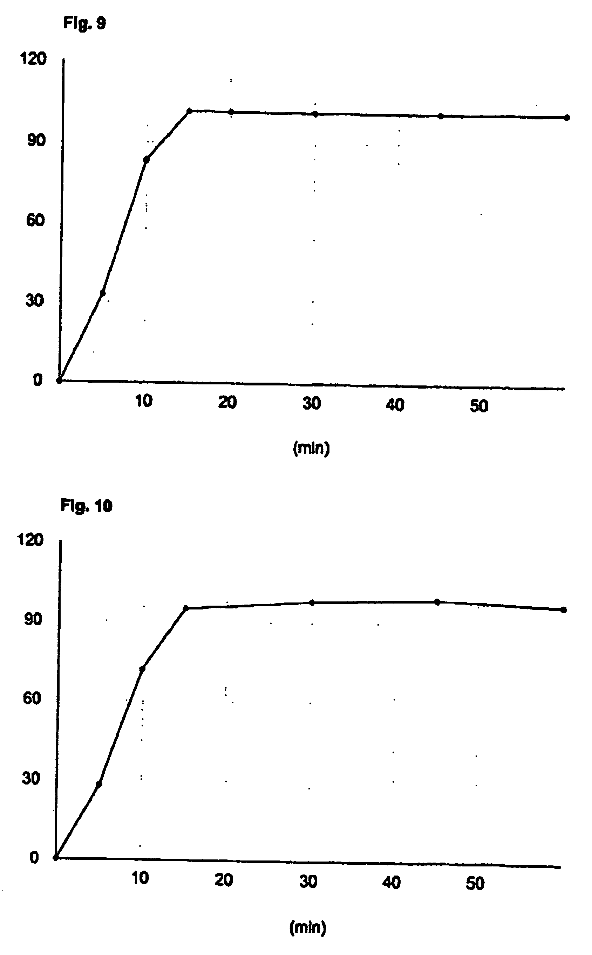

Fig. 9 and 10 correspond to dexketoprofen trometamol 12.5 mg dissolution profile (Ex. 3) for pH 6.8 and 1.2, respectively.

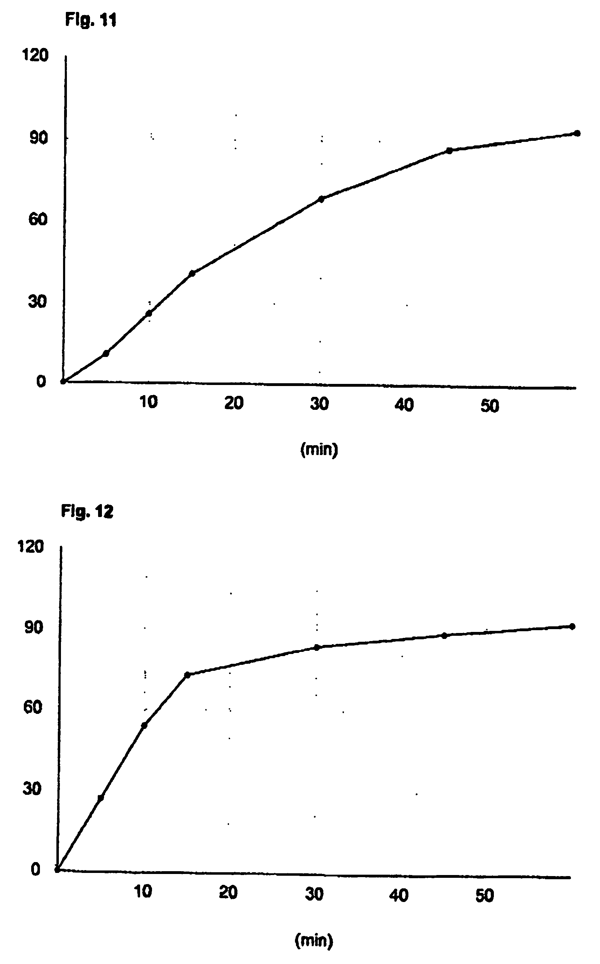

Fig. 11 and 12 correspond to dexketoprofen trometamol 12.5 mg dissolution profile (Ex. 4) for pH 1.0 and pH 6.8, respectively.

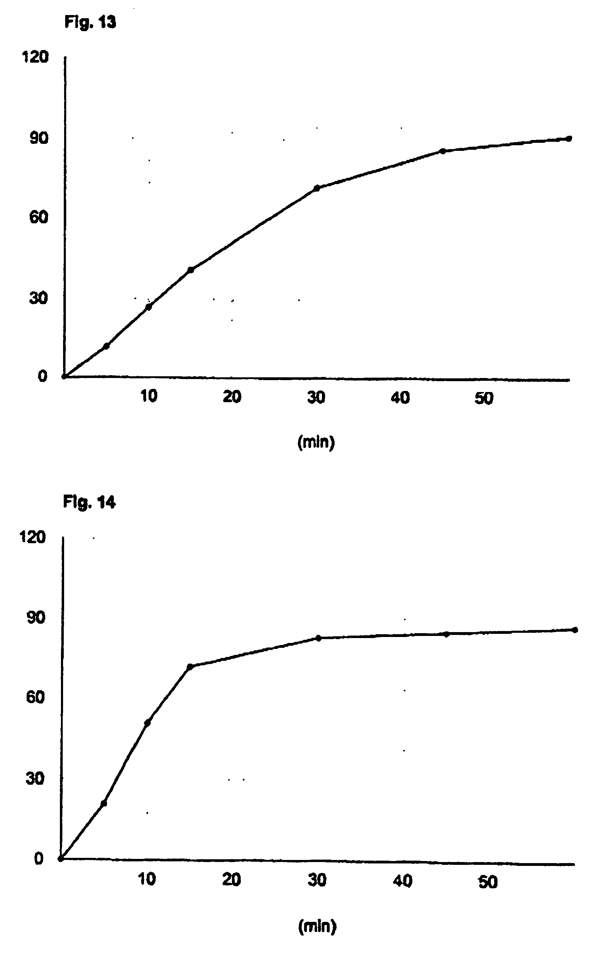

Fig. 13 and 14 correspond to dexketoprofen trometamol 12.5 mg dissolution profile (Ex. 5) for pH 1.0 and pH 6.8, respectively.

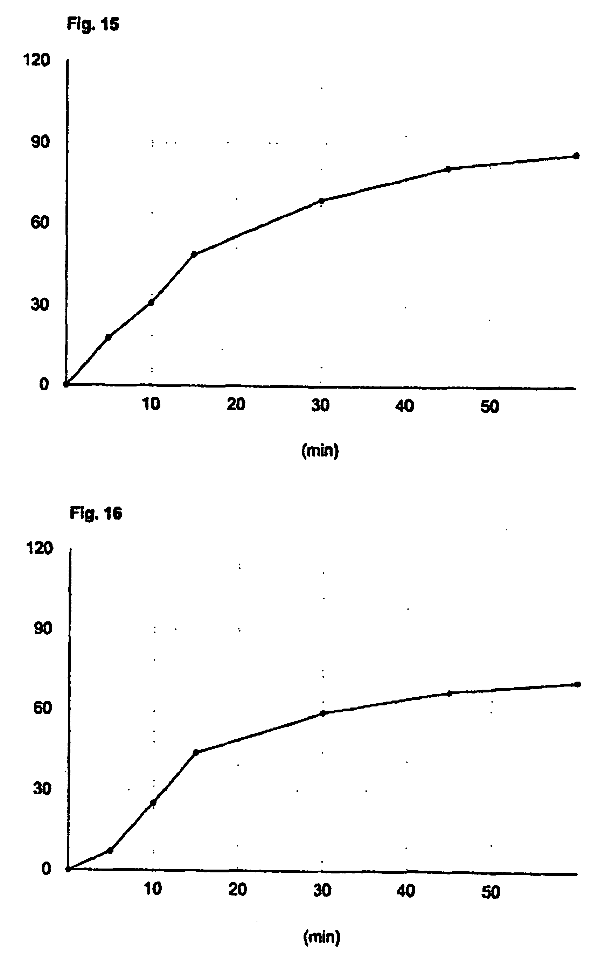

Fig. 15 and 16 correspond to dexketoprofen trometamol 12.5 mg dissolution profile (Ex. 6) for pH 1.0 and pH 6.8, respectively.

EXAMPLES

Example A. Preparation of dexketoprofen trometamol crystalline form C.

A solution of (+)-(S)-2-(3-benzoylphenyl)propionic acid (5.0 g, 19.7 mmol) in ethanol (15 ml) is added with a solution of tromethamine (2.4 g, 19.7 mmol) in water (8 ml). The mixture is stirred at room temperature for 1/2 hour, then evaporated to dryness, to give a semi-solid residue, which is redissolved in ethanol and then evaporated to dryness, to obtain a loose solid which is crystallized from ethanol-ethyl acetate, affording 6.8 g (92 %) of the title compound as a white crystalline solid, with melting point 104.8-105.1°C.

$[alfa]_D^{20}$ =-5.2° (c=1.47, methanol).

IR (KBr): 3060, 1650, 1570, 1400, 1360, 1290, 1020, 720, 650 cm$^{-1}$.

N.M.R. $^1$H (300 MHz, CD$_3$OD) 6 ppm: 1.45 (d, 3H); 3.64 (s, 6H); 3.66 (q,11H); 7.41-7.80 (m, 9H).

Elemental analysis for C$_{20}$H$_{25}$NO$_6$:

Calculated: C, 63.99%: H, 6.71% ; N, 3.73%
Found : C. 63.60% ; H, 6.40%; N, 3.73%

**Example 1**

Dexketoprofen 25.0 mg and 12.5 mg coated tablets:

| Ingredient | Manufacturing formula per tablet 25.0 mg (mg) | Manufacturing formula per tablet 12.5 mg (mg) | Batch formula (100.000 tablets) |
|---|---|---|---|
| Core | | | |
| Dexketoprofen trometamol | 36.90 | 18.45 | 1.845 g |
| Maize starch | 49.6 | 24,80 | 2.480 g |
| Sodium starch glycolate | 27.1 | 13,55 | 1.355 g |
| Microcrystalline cellulose | 141.20 | 70.60 | 7.060 g |
| Glycerol distearate | 5.2 | 2,60 | 260 g |
| - Purified water | q.s | q.s | q.s |
| Core weigth | 260.00 | 130,00 | 1.3000 g |
| Coating | | | |
| Opadry® Y-1-7000 | 2.6 | 2,6 | 2.600 g |

Example 1 Manufacturing Process (batch size 100.000):

1. Sift Dexketoprofen trometamol, sodium starch glicolate, maize starch and microcrystalline cellulose powder through a 18 mesh screen.
2. Mix the sifted material from 2 to 5 min in the granulation machine (pre-mix).
3. Add the binding solution consisting of water and add it for approximately 2 min.
4. Granulate for 10 min the mix components obtained in step 4.
5. Place the sifted material from step 4 into a fluid bed processor bowl and pre-heat for 10 min.
6. Dry the granulate obtained in step 5 until the water content is less than 4 %.
7. Sift the dried granulate through a 10 mm screen.
8. Sift the sodium starch glicolate, cellulose microcristalline through a 1,2 mm screen and then mix these ingredients with the dried granulate 10 min in a V-Blender.
9. Add Glycerol distearate to the final mix of step 8 and blend for 5 min.
10. Compress the lubricated granulate into tablets using 7 x 7 mm cylindrical punches (for the strength of 12,5 mg of dexketoprofen) and 9,2 x 9,2 mm cylindrical punches (for the strength of 25 mg).
11. Final step consists of protecting the cores with a fiilm-coating polymer using a solution, such as one comprising hydroxypropyl methylcellulose (1 %), polyethylene glycol 400 (1 %), and titanium dioxide (0.05 %) in suitable equipment, such as pan coater equipment.

**Examples 2 and 3**

Examples 2 and 3 correspond to two additional industrial batches of dexketoprofen trometamol tablets prepared as in example 1.

Granulate properties have been characterized in order to classify the granulate obtained in example 1, 2 and 3.

Carr's index and the Hausner ratio are both based on the decrease in powder volume during tapping, and their usefulness lies in their ability to predict compressibility and flowability. The lower the number, the more free-flowing the powder. An increase in the value is proportional to adhesion and friction properties of a powder, including (attractive) triboelectric charge. The mathematical expression of each one is:

$$\text{Carr's Index (Compressibility Index) } 100^*(V_o - V_f)/V_o = 100''(D_f - D_o)/D_f$$

$$\text{Hausner Ratio } V_oM = D_f/D_o$$

| Angle of repose | Carr's Index | Fluidity | Hausner ratio |
|---|---|---|---|
| 25 - 30 | 0 - 10 | Excellent | 1.00 - 1.11 |
| 31 - 35 | 11 - 15 | Very good | 1.12 - 1.18 |
| 36 - 40 | 16 - 20 | Good | 1.19 - 1.25 |
| 41 - 45 | 21 - 25 | Tolerable | 1.26 - 1.34 |
| 46 - 55 | 26 - 31 | Poor | 1.35 - 1.45 |
| 56 - 65 | 32 - 37 | Very poor | 1.46 - 1.59 |
| > 66 | > 38 | Extremely poor | > 1.60 |

Table 1. Characteristics of dexketoprofen granulates prepared by example 1, 2 and 3.

| Example | Powder cone | Hausner ratio | Carr's Index |
|---|---|---|---|
| 1 (strength 12.5 mg) | 27.5 | 1.33 | 25% |
| 2 (strength 12.5 mg) | 28.2 | 1.33 | 25% |
| 3 (strength 12.5 mg) | 32.8 | 1.26 | 21% |
| Fluidity | Excellent | Tolerable | Tolerable |

Table 2. Residual water content in the granulate:

| Example | Water content (%) |
|---|---|
| 1 (strength 12,5 mg) | 4.8 |
| 2 (strength 12,5 mg) | 3.3 |
| 3 (strength 12,5 mg) | 3.3 |

Table 3. Pharmacotechnical parameters of the tablets (strength 12,5 mg)

| Example | Mean weight (mg) | Thickness (mm) | Hardness (N) | Friability (%) |
|---|---|---|---|---|
| 1 | 130 | 3.3 | 53 | 0.0 |
| 2 | 130 | 3.3 | 48 | 0.0 |
| 3 | 130 | 3.32 | 67 | 0.0 |

Dissolution profile of the prepared tablets. (strength 12,5 mg)
**Dissolution conditions (pH=6,8)**

| Time (min) | Example 1 | Example 2 | Example 3 |
|---|---|---|---|
| 0 | 0,0% | 0,0% | 0,0% |
| 5 | 36,0% | 41,9% | 33,2% |
| 10 | 92,0% | 91,8% | 83,4% |
| 15 | 98,1% | 103,7% | 101,5% |
| 20 | 98,1% | 103,9% | 101,6% |
| 30 | 97,8% | 103.8% | 101,5% |
| 45 | 97,8% | 104,0% | 101,5% |
| 60 | 97,7% | 104,0% | 101,6% |

Dissolution profile at pH 1.2 for example 1, 2 and 3
Ex. 1 dexketoprofen trometamol 12.5 mg dissolution-profile for pH 1.2.

| TIME | V1 | V2 | V3 | V4 | V5 | V6 | V7 | V8 |
|---|---|---|---|---|---|---|---|---|
| 0 min | 0.00 | 0.00 | 0.00 | 0.00 | 0.00 | 0.00 | 0.00 | 0.00 |
| 5 min | 28.14 | 21.65 | 18.18 | 25.54 | 21.86 | 9.09 | 21.08 | 22.58 |
| 10 min | 72.08 | 58.44 | 56.71 | 66.23 | 56.49 | 38.10 | 74.07 | 73.35 |
| 15 min | 93.29 | 87.88 | 87.45 | 92.21 | 85.50 | 80.30 | 88.38 | 94.44 |
| 30 min | 97.84 | 95.24 | 93.94 | 98.05 | 96.97 | 95.24 | 92.12 | 99.29 |
| 45 min | 98.27 | 97.40 | 94.16 | 98.70 | 97.84 | 96.54 | 92.49 | 99.81 |
| 60 min | 98.27 | 97.40 | 94.59 | 98.70 | 97.84 | 96.10 | 92.71 | 99.89 |

| TIME | V9 | V10 | V11 | V12 | Average | SD | RSD |
|---|---|---|---|---|---|---|---|
| 0 min | 0.00 | 0.00 | 0.00 | 0.00 | 0.00 | 0.00 | 0.00% |
| 5 min | 33.87 | 26.86 | 16.06 | 31.49 | 23.03 | 6.81 | 29.56% |

(continued)

| TIME | V9 | V10 | V11 | V12 | Average | SD | RSD |
|---|---|---|---|---|---|---|---|
| 10 min | 80.76 | 67.86 | 52.68 | 69.31 | 63.84 | 11.76 | 18.43% |
| 15 min | 96.43 | 88.64 | 87.99 | 91.75 | 89.52 | 4.38 | 4.89% |
| 30 min | 100.00 | 94.33 | 97.97 | 96.95 | 96.49 | 2.34 | 2.43% |
| 45 min | 100.22 | 94.85 | 98.38 | 97.14 | 97.15 | 2.31 | 2.37 % |
| 60 min | 100.35 | 95.04 | 98.55 | 97.47 | 97.24 | 2.25 | 2.31% |

Ex. 2 dexketoprofen trometamol 12.5 mg dissolution profile for pH 1.2.

| TIME | V1 | V2 | V3 | V4 | V5 | V6 | V7 | V8 |
|---|---|---|---|---|---|---|---|---|
| 0 min | 0.00 | 0.00 | 0.00 | 0.00 | 0.00 | 0.00 | 0.00 | 0.00 |
| 5 min | 38.20 | 24.65 | 38.05 | 45.97 | 35.28 | 35.97 | 31.47 | 31.54 |
| 10 min | 89.87 | 69.44 | 79.65 | 86.56 | 72.66 | 79.74 | 73.01 | 76.56 |
| 15 min | 103.12 | 93.51 | 98.14 | 102.51 | 96.32 | 100.67 | 95.84 | 99.46 |
| 30 min | 106.26 | 99.48 | 103.81 | 106.23 | 101.04 | 104.68 | 100.76 | 103.48 |
| 45 min | 106.54 | 100.15 | 104.20 | 106.58 | 101.62 | 105.11 | 101.02 | 103.83 |
| 60 min | 106.86 | 100.52 | 104.59 | 106.64 | 102.08 | 105.26 | 101.26 | 103.96 |

| TIME | V9 | V10 | V11 | V12 | Average | SD | RSD |
|---|---|---|---|---|---|---|---|
| 0 min | 0.00 | 0.00 | 0.00 | 0.00 | 0.00 | 0.00 | 0.00 % |
| 5 min | 38.87 | 37.32 | 33.31 | 31.58 | 35.19 | 5.28 | 15.02 % |
| 10 min | 87.90 | 77.23 | 82.45 | 75.19 | 79.19 | 6.45 | 8.15 % |
| 15 min | 101.19 | 99.18 | 100.56 | 98.96 | 99.12 | 2.82 | 2.84 % |
| 30 min | 104.00 | 105.58 | 103.98 | 104.29 | 103.63 | 2.18 | 2.09 % |
| 45 min | 104.35 | 105.97 | 104.20 | 104.59 | 104.01 | 2.09 | 2.01 % |
| 60 min | 104.59 | 105.82 | 104.20 | 104.87 | 104.24 | 2.03 | 1.95 % |

Ex. 3 dexketoprofen trometamol 12.5 mg dissolution profile for pH 1.2.

| TIME | Average |
|---|---|
| 0 min | 0 |
| 5 min | 28 |
| 10 min | 72 |
| 15 min | 95 |
| 30 min | 98 |
| 45 min | 99 |
| 60 min | 97 |

**Examples 4, 5 and 6.**

| Ingredients | Ex. 1 (%) | Ex. 2 (%) | Ex. 3 (%) |
|---|---|---|---|
| Dexketo. trometamol | 14.19 | 14.19 | 14.19 |
| Maize starch | -- | -- | 13.00 |
| Pregelatinized maize starch | 34.00 | 26.00 | 13.00 |
| Cel microcryst pH 102 | 44.31 | 52.31 | 52.31 |
| HPMC K100M | 6.00 | 6.00 | 6.00 |
| HPMC K4M | -- | -- | -- |
| Colloidal silica | 0.5 | 0.5 | 0.5 |
| Magnesium stearate | 1.0 | 1.00 | 1.00 |
| Coating polymer | 2.00 | 2.00 | 2.00 |

Manufacturing process of the tablets of example 4, 5 and 6 (batch size 100.000)

Dexketoprofen and the rest of the excipients of the formulation were blended for 10 minutes in a blender. The mixture was screened through 1 mm mesh sieve. After that, the magnesium stearate were added and blended for 10 minutes. Then the mixture was compressed to form tablets using a rotary trabletting machine, equipped with 7 x 7 mm for the strengh of 12,5 mg of Dexketoprofen and 9.2 x 9,2 mm for the strenght of 25 mg cilindrical punches.

Final step consist of protecting tablets with a film-coating polymer by using for instance a solution, such as one comprising hydroxypropyl methylcellulose (1 %), polyethylene glycol 400 (1 %), and titanium dioxide (0.05 %) in suitable equipment, such as pan coater equipment.

Ex. 4 dexketoprofen trometamol 12.5 mg dissolution profile for pH 1.0 and pH 6.8, respectively.

| TIME | pH 1.0 | pH 6.8 |
|---|---|---|
| 0 min | 0 | 0 |
| 5 min | 27 | 11 |
| 10 min | 54 | 26 |
| 15 min | 73 | 41 |
| 30 min | 84 | 69 |
| 45 min | 89 | 87 |
| 60 min | 93 | 94 |

Ex. 5 dexketoprofen trometamol 12.5 mg dissolution profile for pH 1.0 and pH 6.8, respectively.

| TIME | pH 1.0 | pH 6.8 |
|---|---|---|
| 0 min | 0 | 0 |
| 5 min | 21 | 12 |
| 10 min | 51 | 27 |
| 15 min | 72 | 41 |
| 30 min | 83 | 72 |
| 45 min | 85 | 86 |
| 60 min | 87 | 91 |

Ex. 6 dexketoprofen trometamol 12.5 mg dissolution profile for pH 1.0 and pH 6.8, respectively.

| TIME | pH 1.0 | pH 6.8 |
|--------|--------|--------|
| 0 min | 0 | 0 |
| 5 min | 7 | 18 |
| 10 min | 25 | 31 |
| 15 min | 44 | 49 |
| 30 min | 59 | 69 |

| 45 min | 67 | 81 |
|--------|--------|--------|
| 60 min | 71 | 86 |

Table 5: Technical parameters of tablets. (strength 12.5 mg)

| LOTE | Ex. 4 | Ex. 5 | Ex. 6 | Average | Max. Diff. |
|--------|--------|--------|--------|--------|--------|
| Granulate water content | 5.1 % | 5.2 % | 5.4 % | 5.2 % | 0.3% |
| Residual water content | 5.3% | 5.0% | 5.1 % | 5.2% | 0.4 % |
| Core hardness (N) | 98 N | 105 N | 102 N | 102 N | 7N |
| C.T. hardness (N) | 128 N | 129 N | 114 N | 123 N | 15 N |
| Core disintegration | 9 min 30 s | 9 min 25 s | 8 min 42 s | 9 min 12 s | 48 s |
| C.T. disintegration | 8 min 54 s | 8 min 23 s | 7min18s | 8 min 11 s | 1 min 36 s |
| Core friability (%) | 0.0% | 0.1 % | 0.1% | 0.1 % | 0% |

**Claims**

1. A process for the manufacture of an immediate release oral pharmaceutical composition comprising dexketoprofen trometamol in the form of tablets or granules, wherein the pharmaceutical composition comprises between 3 % and 60 % by weight of dexketoprofen trometamol in respect of the total amount of the pharmaceutical composition, wherein the total amount of lubricant or lubricants present in the pharmaceutical composition ranges from 0.5 to 6 % by weight in respect of the total amount of the pharmaceutical composition; wherein the total amount of disintegrant or disintegrants present in the pharmaceutical composition ranges from 2 to 20 % by weight in respect of the total amount of the pharmaceutical composition; wherein the total amount of diluent or diluents present in the pharmaceutical composition ranges from 15 % to 85 % by weight in respect of the total amount of the pharmaceutical composition, wherein the process for the manufacture of the pharmaceutical composition comprises the steps of:

   i) sieving when appropriate the intra-granular ingredients to form uniformly sized particles of intra-granular ingredients;
   ii) either mixing the particles of intra-granular ingredients and dexketoprofen trometamol, preferably in a granulator-mixer; granulate the obtained mixture with at least one binder, preferably the binder comprises water, more preferably consists essentially of water; even more preferably consists of water; wherein the final water content of the granulate is from about 5 % to about 30 % by weight;
   iii) or mixing the particles of intra-granular ingredients, preferably in a granulator-mixer; granulate the obtained mixture with a solution or dispersion of dexketoprofen trometamol in water; wherein the final water content of the granulate is from about 5 % to about 30 % by weight;
   iv) optionally and/or if required, performing a sieving of the granulate obtained in either step (ii) or step (iii);
   v) drying the granulate of the previous step (ii), (iii) or (iv) to an endpoint water content of less than about 8 % by weight, preferably less than 6.5 by weight, more preferably from about 1.0 % to 5.5 % by weight, wherein the drying is preferably carried out in a fluid bed at an input temperature between 35 and 105 °C;
   vi) mixing the granulate of the previous step with the extra-granular agents and blending to form a blend;

vii) and, when the pharmaceutical composition is in the form of tablets, compressing the blend into tablets using a tablet press.

2. The process according to the preceding claims, wherein the pharmaceutical composition comprises between a 10 % and a 20 % by weight of dexketoprofen trometamol in respect of the total amount of the pharmaceutical composition.

3. The process according to any one of the preceding claims, wherein the total amount of lubricant or lubricants present in the pharmaceutical composition ranges from 1.2 to 3.5 % by weight and/or wherein the pharmaceutical comprises magnesium stearate, glycerol distearate, stearic acid or mixtures thereof, more preferably the pharmaceutical composition comprises glycerol distearate.

4. The process according to any one of the preceding claims, wherein the total amount of disintegrant or disintegrants present in the pharmaceutical composition ranges from 4 to 16 % by weight and/or wherein the pharmaceutical comprises at least a disintegrant selected from water-soluble disintegrants, such as pregelatinized starch, sodium carboxymethyl cellulose and sodium alginate, more preferably the pharmaceutical composition comprises sodium carboxymethyl cellulose.

5. The process according to any one of the preceding claims, wherein the total amount of diluent or diluents present in the pharmaceutical composition ranges from 35 % to 65 % by weight, wherein the total amount of diluent or diluents present in the pharmaceutical composition ranges from 45 % 60 % by weight and/or wherein the pharmaceutical comprises at least a diluent, such as lactose, calcium phosphate and microcrystalline cellulose, more preferably the pharmaceutical composition comprises microcrystalline cellulose.

6. The process according to any one of the preceding claims, wherein the final water content of the granulate of step (ii) or (iii) is from 14 % to 21 % by weight or wherein in step (v) the granulate of the previous step (ii), (iii) or (iv) is dried to an endpoint water content from 1.5% to 4.5 % by weight or wherein in step (v) the granulate of the previous step (ii), (iii) or (iv) is dried to an endpoint water content from 1.6 % to 3.5 % by weight or wherein in step (v) the drying of the granulate is carried out in a fluid bed at an input temperature between 40 and 80 °C.

7. The process according to any one of the preceding claims, wherein the pharmaceutical composition is in the form of tablets.

8. The process according to any one of the preceding claims, wherein the dexketoprofen trometamol used in step (ii) or the dexketoprofen trometamol used to form the solution or dispersion of step (iii) is in a crystalline, amorphous form or mixtures thereof, wherein the dexketoprofen trometamol used in step (ii) is in a crystalline form A, B, C or mixtures thereof, wherein the dexketoprofen trometamol used in step (ii) is dexketoprofen trometamol crystalline form A and/or C or wherein the dexketoprofen trometamol used in step (ii) or the dexketoprofen trometamol used to form the solution or dispersion of step (iii) has an average particle size from 5 to 90 microns as measured by laser light diffraction.

9. The process according to any one of the preceding claims, wherein the pharmaceutical composition comprises between 33 and 40 mg of dexketoprofen trometamol by unit dosis.

10. The immediate release oral pharmaceutical composition in the form of tablets or granules, preferably for the symptomatic treatment of pain of mild to moderate intensity, such a musculo-skeletal pain, dysmenorrhoea or dental pain, comprising the active ingredient as defined in any one of the claims 1 to 9, wherein the pharmaceutical composition is manufactured by any one of the processes as defined in any one of the preceding process claims.

11. A immediate release granulate comprising between 3 % and 60 % by weight of dexketoprofen trometamol in respect of the total amount of the granule, at least one disintegrant and at least one diluent or wherein the granulate comprises between a 5 % and a 45 % by weight of dexketoprofen trometamol in respect of the total amount of the granulate.

12. A process for the manufacture of the granulate as defined in claim 11, wherein the process comprises the following steps:

    i) sieving when appropriate the intra-granular ingredients to form uniformly sized particles of intra-granular ingredients;
    ii) either mixing the particles of intra-granular ingredients and dexketoprofen trometamol, preferably in a granulator-mixer; granulate the obtained mixture with at least one binder, preferably the binder comprises water, more preferably consists essentially of water; even more preferably consists of water; wherein the final water content of the granulate is from about 5 % to about 30 % by weight;

iii) or mixing the particles of intra-granular ingredients, preferably in a granulator-mixer; granulate the obtained mixture with a solution or dispersion of dexketoprofen trometamol in water; wherein the final water content of the granulate is from about 5 % to about 30 % by weight;

iv) optionally and/or if required, performing a sieving of the granulate obtained in either step (ii) or step (iii);

v) drying the granulate of the previous step (ii), (iii) or (iv) to an endpoint water content of less than about 8 % by weight, preferably less than 6.5 by weight, more preferably from about 1.0 % to 5.5 % by weight, wherein the drying is preferably carried out in a fluid bed at an input temperature between 35 and 105 °C.

13. An immediate release oral pharmaceutical composition comprising the granulate as defined in claim 11 or an oral pharmaceutical composition according to claim 10 comprising the granulate of claim 11.

14. A powder comprising the granulate as defined in claim 11.

15. Use of a granulate according to any of the claim 11 or the powder of claim 14 for the manufacture of an oral pharmaceutical composition, preferebly a tablet, more preferably coated tablet.

**Patentansprüche**

1. Verfahren zur Herstellung einer oralen pharmazeutischen Zusammensetzung mit sofortiger Freisetzung, die Dexketoprofen-Trometamol in Form von Tabletten oder Granulaten umfasst, wobei die pharmazeutische Zusammensetzung zwischen 3 Gew.-% und 60 Gew.-% an Dexketoprofen-Trometamol in Bezug auf die Gesamtmenge der pharmazeutischen Zusammensetzung umfasst, wobei die Gesamtmenge des oder der in der pharmazeutischen Zusammensetzung vorhandenen Gleitmittels oder Gleitmittel im Bereich von 0,5 bis 6 Gew.-% in Bezug auf die Gesamtmenge der pharmazeutischen Zusammensetzung liegt; wobei die Gesamtmenge des oder der in der pharmazeutischen Zusammensetzung vorhandenen Sprengmittels oder Sprengmittel im Bereich von 2 Gew.-% bis 20 Gew.-% in Bezug auf die Gesamtmenge der pharmazeutischen Zusammensetzung liegt; wobei die Gesamtmenge des oder der in der pharmazeutischen Zusammensetzung vorhandenen Verdünnungsmittels oder Verdünnungsmittel im Bereich von 15 Gew.-% bis 85 Gew.-% in Bezug auf die Gesamtmenge der pharmazeutischen Zusammensetzung liegt, wobei das Verfahren zur Herstellung der pharmazeutischen Zusammensetzung die folgenden Schritte umfasst:

i) gegebenenfalls Sieben der intragranulären Bestandteile, um einheitlich klassierte Partikel intragranulärer Bestandteile zu bilden;

ii) entweder Mischen der Partikel intragranulärer Bestandteile und von Dexketoprofen-Trometamol, vorzugsweise in einem Granulator-Mischer; Granulieren des gewonnenen Gemischs mit mindestens einem Bindemittel, wobei das Bindemittel bevorzugt Wasser umfasst, noch bevorzugter im Wesentlichen aus Wasser besteht; und am bevorzugsten aus Wasser besteht; wobei der endgültige Wassergehalt des Granulats zwischen ungefähr 5 Gew.-% und ungefähr 30 Gew.-% beträgt;

iii) oder Mischen der Partikel intragranulärer Bestandteile, vorzugsweise in einem Granulator-Mischer; Granulieren des gewonnenen Gemischs mit einer Lösung oder Dispersion von Dexketoprofen-Trometamol in Wasser; wobei der endgültige Wassergehalt des Granulats zwischen ungefähr 5 Gew.-% und ungefähr 30 Gew.-% beträgt;

iv) wahlweise und/oder falls erforderlich Durchführen einer Siebung des entweder in Schritt (ii) oder in Schritt (iii) gewonnenen Granulats;

v) Trocknen des Granulats des vorausgehenden Schritts (ii), (iii) oder (iv) zu einem Endpunkt-Wassergehalt von weniger als ungefähr 8 Gew.-%, bevorzugt von weniger als 6,5 Gew.-%, noch bevorzugter zwischen ungefähr 1,0 Gew.-% und 5,5 Gew.-%, wobei die Trocknung vorzugsweise in einem Fließbett bei einer Eingangstemperatur zwischen 35 und 105 °C durchgeführt wird;

vi) Mischen des Granulats des vorausgehenden Schritts mit den extragranulären Mitteln und Vermengen, um ein Gemisch zu bilden;

vii) sowie, falls die pharmazeutische Zusammensetzung Tablettenform hat, Komprimieren des Gemischs in Tabletten unter Verwendung einer Tablettenpresse.

2. Verfahren nach vorausgehenden Ansprüchen, wobei die pharmazeutische Zusammensetzung zwischen 10 Gew.-

% und 20 Gew.-% an Dexketoprofen-Trometamol in Bezug auf die Gesamtmenge der pharmazeutischen Zusammensetzung umfasst.

3. Verfahren nach einem der vorausgehenden Ansprüche, wobei die Gesamtmenge des oder der in der pharmazeutischen Zusammensetzung vorhandenen Gleitmittels oder Gleitmittel im Bereich von 1,2 bis 3,5 Gew.-% liegt und/oder wobei das Arzneimittel Magnesiumstearat, Glycerindistearat, Stearinsäure oder Mischungen davon umfasst, wobei die pharmazeutische Zusammensetzung noch bevorzugter Glycerindistearat umfasst.

4. Verfahren nach einem der vorausgehenden Ansprüche, wobei die Gesamtmenge des oder der in der pharmazeutischen Zusammensetzung vorhandenen Sprengmittels oder Sprengmittel im Bereich von 4 bis 16 Gew.-% liegt und/oder wobei das Arzneimittel mindestens ein aus wasserlöslichen Sprengmitteln ausgewähltes Sprengmittel wie beispielsweise vorgelatinierte Stärke, Natrium-Carboxymethylcellulose oder Natriumalginat umfasst, wobei die pharmazeutische Zusammensetzung noch bevorzugter Natrium-Carboxymethylcellulose umfasst.

5. Verfahren nach einem der vorausgehenden Ansprüche, wobei die Gesamtmenge des oder der in der pharmazeutischen Zusammensetzung vorhandenen Verdünnungsmittels oder Verdünnungsmittel im Bereich von 35 bis 65 Gew.-% liegt, wobei die Gesamtmenge des oder der in der pharmazeutischen Zusammensetzung vorhandenen Verdünnungsmittels oder Verdünnungsmittel in einem Bereich von 45 bis 60 Gew.-% liegt und/oder wobei das Arzneimittel mindestens ein Verdünnungsmittel wie beispielsweise Lactose, Calciumphosphat oder mikrokristalline Cellulose umfasst, wobei die pharmazeutische Zusammensetzung noch bevorzugter mikrokristalline Cellulose umfasst.

6. Verfahren nach einem der vorausgehenden Ansprüche, wobei der endgültige Wassergehalt des Granulats von Schritt (ii) oder (iii) zwischen 14 Gew.-% und 21 Gew.-% liegt oder wobei in Schritt (v) das Granulat der vorausgehenden Schritte (ii), (iii) oder (iv) zu einem Endpunkt-Wassergehalt von zwischen 1,5 Gew.-% und 4,5 Gew.-% getrocknet wird oder wobei in Schritt (v) das Granulat der vorausgehenden Schritte (ii), (iii) oder (iv) zu einem Endpunkt-Wassergehalt von zwischen 1,6 Gew.-% und 3,5 Gew.-% getrocknet wird oder wobei in Schritt (v) das Trocknen des Granulats in einem Fließbett bei einer Eingangstemperatur von zwischen 40 und 80 °C durchgeführt wird.

7. Verfahren nach einem der vorausgehenden Ansprüche, wobei die pharmazeutische Zusammensetzung die Form von Tabletten hat.

8. Verfahren nach einem der vorausgehenden Ansprüche, wobei das in Schritt (ii) verwendete Dexketoprofen-Trometamol oder das zur Bildung der Lösung oder Dispersion von Schritt (iii) verwendete Dexketoprofen-Trometamol in einer kristallinen, amorphen Form oder einer Mischung davon vorliegt, wobei das in Schritt (ii) verwendete Dexketoprofen-Trometamol in einer kristallinen Form A, B, C oder einer Mischung davon vorliegt, wobei das in Schritt (ii) verwendete Dexketoprofen-Trometamol Dexketoprofen-Trometamol in der kristallinen Form A und/oder C ist oder wobei das in Schritt (ii) verwendete Dexketoprofen-Trometamol oder das zur Bildung der Lösung oder Dispersion von Schritt (iii) verwendete Dexketoprofen-Trometamol eine durchschnittliche Partikelgröße von zwischen 5 und 90 $\mu$m aufweist, gemessen mittels Laserlichtbeugung.

9. Verfahren nach einem der vorausgehenden Ansprüche, wobei die pharmazeutische Zusammensetzung zwischen 33 und 40 mg Dexketoprofen-Trometamol pro Einheitsdosis umfasst.

10. Orale pharmazeutische Zusammensetzung mit sofortiger Freisetzung in Form von Tabletten oder Granulaten, vorzugsweise für die symptomatische Behandlung von Schmerzen von leichter bis mittlerer Intensität wie beispielsweise Muskel-Skelett-Schmerzen, Dysmenorrhö oder Zahnschmerzen, den wie in einem der Ansprüche 1 bis 9 definierten Wirkstoff umfassend, wobei die pharmazeutische Zusammensetzung durch eines der wie in einem der vorausgehenden Ansprüchen definierten Verfahren hergestellt wird.

11. Ein Granulat mit sofortiger Freisetzung, zwischen 3 Gew.-% und 60 Gew.-% Dexketoprofen-Trometamol in Bezug auf die Gesamtmenge des Granulats, mindestens ein Sprengmittel und mindestens ein Verdünnungsmittel umfassend, oder wobei das Granulat zwischen 5 Gew.-% und 45 Gew.-% Dexketoprofen-Trometamol in Bezug auf die Gesamtmenge des Granulats umfasst.

12. Verfahren zur Herstellung des wie in Anspruch 11 definierten Granulats, wobei das Verfahren die folgenden Schritte umfasst:

i) gegebenenfalls Sieben der intragranulären Bestandteile, um einheitlich klassierte Partikel intragranulärer Bestandteile zu bilden;

ii) entweder Mischen der Partikel intragranulärer Bestandteile und von Dexketoprofen-Trometamol, vorzugsweise in einem Granulator-Mischer; Granulieren des gewonnenen Gemischs mit mindestens einem Bindemittel, wobei das Bindemittel bevorzugt Wasser umfasst, noch bevorzugter im Wesentlichen aus Wasser besteht; und am bevorzugsten aus Wasser besteht; wobei der endgültige Wassergehalt des Granulats zwischen ungefähr 5 Gew.-% und ungefähr 30 Gew.-% beträgt;

iii) oder Mischen der Partikel intragranulärer Bestandteile, vorzugsweise in einem Granulator-Mischer; Granulieren des gewonnenen Gemischs mit einer Lösung oder Dispersion von Dexketoprofen-Trometamol in Wasser; wobei der endgültige Wassergehalt des Granulats zwischen ungefähr 5 Gew.-% und ungefähr 30 Gew.-% beträgt.

iv) wahlweise und/oder falls erforderlich Durchführen einer Siebung des entweder in Schritt (ii) oder in Schritt (iii) gewonnenen Granulats;

v) Trocknen des Granulats des vorausgehenden Schritts (ii), (iii) oder (iv) bis zu einem Endpunkt-Wassergehalt von weniger als ungefähr 8 Gew.-%, bevorzugt von weniger als 6,5 Gew.-%, noch bevorzugter zwischen ungefähr 1,0 Gew.-% und 5,5 Gew.-%, wobei die Trocknung vorzugsweise in einem Fließbett bei einer Eingangstemperatur von zwischen 35 und 105 °C durchgeführt wird.

13. Orale pharmazeutische Zusammensetzung mit sofortiger Freisetzung, das wie in Anspruch 11 definierte Granulat umfassend, oder orale pharmazeutische Zusammensetzung nach Anspruch 10, das Granulat nach Anspruch 11 umfassend.

14. Pulver, das wie in Anspruch 11 definierte Granulat umfassend.

15. Verwendung eines Granulats nach einem der Ansprüche 11 oder Pulver nach Anspruch 14 zur Herstellung einer oralen pharmazeutischen Zusammensetzung, bevorzugt einer Tablette, noch bevorzugter einer Filmtablette.

**Revendications**

1. Procédé de fabrication d'une composition pharmaceutique orale à libération immédiate comprenant du dexkétoprofène trométamol sous la forme de comprimés ou de granulés, dans lequel la composition pharmaceutique comprend entre 3 % et 60 % en poids de dexkétoprofène trométamol par rapport à la quantité totale de la composition pharmaceutique, dans lequel la quantité totale de lubrifiant ou de lubrifiants présente dans la composition pharmaceutique va de 0,5 à 6 % en poids par rapport à la quantité totale de la composition pharmaceutique, dans lequel la quantité totale de délitant ou de délitants présente dans la composition pharmaceutique va de 2 à 20 % en poids par rapport à la quantité totale de la composition pharmaceutique ; dans lequel la quantité totale de diluant ou de diluants présente dans la composition pharmaceutique va de 15 % à 85 % en poids par rapport à la quantité totale de la composition pharmaceutique, le procédé de fabrication de la composition pharmaceutique comprenant les étapes suivantes:

i) le tamisage si nécessaire des ingrédients intragranulaires pour former des particules d'ingrédients intragranulaires de taille uniforme;

ii) soit le mélange des particules d'ingrédients intragranulaires et de dexkétoprofène trométamol, de préférence dans un granulateur-mélangeur ; la granulation du mélange obtenu avec au moins un liant, le liant comprenant de préférence de l'eau, étant plus préférablement essentiellement constitué d'eau ; étant encore plus préférablement constitué d'eau; dans lequel la teneur en eau finale du granulat est d'environ 5 % à environ 30 % en poids;

iii) soit le mélange des particules d'ingrédients intragranulaires, de préférence dans un granulateur-mélangeur ; la granulation du mélange obtenu avec une solution ou dispersion de dexkétoprofène trométamol dans de l'eau ; dans lequel la teneur en eau finale du granulat est d'environ 5 à environ 30 % en poids;

iv) la réalisation optionnelle et/ou si nécessaire d'un tamisage du granulat obtenu dans l'une ou l'autre de l'étape (ii) ou de l'étape (iii);

v) le séchage du granulat de l'étape (ii), (iii) ou (iv) précédente jusqu'à une teneur en eau finale inférieure à environ 8 % en poids, de préférence, inférieure à 6,5 % en poids, mieux encore, d'environ 1,0 % à 5,5 % en poids, le séchage étant de préférence effectué dans un lit fluide à une température d'entrée comprise entre 35 et 105°C;

vi) la mixtion du granulat de l'étape précédente avec les agents extragranulaires et le mélangeage pour former un mélange;

vii) et, lorsque la composition pharmaceutique est sous la forme de comprimés, la compression du mélange en comprimés au moyen d'une presse à comprimés.

2. Procédé selon la revendication précédente, dans lequel la composition pharmaceutique comprend entre 10 % et 20 % en poids de dexkétoprofène trométamol par rapport à la quantité totale de la composition pharmaceutique.

3. Procédé selon l'une quelconque des revendications précédentes, dans lequel la quantité totale de lubrifiant ou de lubrifiants présente dans la composition pharmaceutique va de 1,2 à 3,5 % en poids et/ou dans lequel la composition pharmaceutique comprend du stéarate de magnésium, du distéarate de glycérol, de l'acide stéarique ou des mélanges de ceux-ci, plus préférablement, la composition pharmaceutique comprend du distéarate de glycérol.

4. Procédé selon l'une quelconque des revendications précédentes, dans lequel la quantité totale de délitant ou de délitants présente dans la composition pharmaceutique va de 4 à 16 % en poids et/ou dans lequel la composition pharmaceutique comprend au moins un délitant choisi parmi les délitants hydrosolubles, tels que de l'amidon prégélatinisé, de la cellulose de carboxyméthyle de sodium et de l'alginate de sodium, plus préférablement, la composition pharmaceutique comprend de la cellulose de carboxyméthyle de sodium.

5. Procédé selon l'une quelconque des revendications précédentes, dans lequel la quantité totale de diluant ou de diluants présente dans la composition pharmaceutique va de 35 % à 65 % en poids, dans lequel la quantité totale de diluant ou de diluants présente dans la composition pharmaceutique va de 45 % à 60 % en poids et/ou dans lequel la composition pharmaceutique comprend au moins un diluant , tel que du lactose, du phosphate de calcium et de la cellulose microcristalline, plus préférablement, la composition pharmaceutique comprend de la cellulose microcristalline.

6. Procédé selon l'une quelconque des revendications précédentes, dans lequel la teneur en eau finale du granulat de l'étape (ii) ou (iii) est de 14 % à 21 % en poids ou dans lequel à l'étape (v), le granulat de l'étape (ii), (iii) ou (iv) précédente est séché à une teneur en eau finale de 1,5 % à 4,5 % en poids ou dans lequel à l'étape (v), le granulat de l'étape (ii), (iii) ou (iv) précédente est séché à une teneur en eau finale de 1,6 % à 3,5 % en poids ou dans lequel à l'étape (v), le séchage du granulat est effectué dans un lit fluide à une température d'entrée comprise entre 40 et 80 °C.

7. Procédé selon l'une quelconque des revendications précédentes, dans lequel la composition pharmaceutique est sous la forme de comprimés.

8. Procédé selon l'une quelconque des revendications précédentes, dans lequel le dexkétoprofène trométamol utilisé à l'étape (ii) ou le dexkétoprofène trométamol utilisé pour former la solution ou dispersion de l'étape (iii) est une forme cristalline, amorphe ou des mélanges de celles-ci, dans lequel le dexkétoprofène trométamol utilisé à l'étape (ii) est sous une forme cristalline A, B, C ou de mélanges de celles-ci, dans lequel le dexkétoprofène trométamol utilisé à l'étape (ii) est une forme cristalline A et/ou C de dexkétoprofène trométamol ou dans lequel le dexkétoprofène trométamol utilisé à l'étape (ii) ou le dexkétoprofène trométamol utilisé pour former la solution ou dispersion de l'étape (iii) a une taille de particule moyenne de 5 à 90 microns telle que mesurée par diffraction de la lumière laser.

9. Procédé selon l'une quelconque des revendications précédentes, dans lequel la composition pharmaceutique comprend entre 33 et 40 mg de dexkétoprofène trométamol par dose unitaire.

10. Composition pharmaceutique orale à libération immédiate sous la forme de comprimés ou de granulés, de préférence pour le traitement symptomatique d'une douleur d'intensité faible à modérée, telle qu'une douleur musculo-squelettique, une dysménorrhée ou une douleur dentaire, comprenant l'ingrédient actif selon l'une quelconque des revendications 1 à 9, la composition pharmaceutique étant fabriquée par l'un quelconque des procédés tels que définis dans l'une quelconque des revendications de procédé précédentes.

11. Granulat à libération immédiate comprenant entre 3 % et 60 % en poids de dexkétoprofène trométamol par rapport à la quantité totale du granulat, au moins un délitant et au moins un diluant ou dans lequel le granulat comprend entre 5 % et 45 % en poids de dexkétoprofène trométamol par rapport à la quantité totale du granulat.

12. Procédé de fabrication du granulat selon la revendication 11, le procédé comprenant les étapes suivantes:

i) le tamisage si nécessaire des ingrédients intragranulaires pour former des particules d'ingrédients intragranulaires de taille uniforme;

ii) soit le mélange des particules d'ingrédients intragranulaires et de dexkétoprofène trométamol, de préférence dans un granulateur-mélangeur ; la granulation du mélange obtenu avec au moins un liant, le liant comprenant de préférence de l'eau, étant plus préférablement essentiellement constitué d'eau ; étant encore plus préférablement constitué d'eau; dans lequel la teneur en eau finale du granulat est d'environ 5 % à environ 30 % en poids;

iii) soit le mélange des particules d'ingrédients intragranulaires, de préférence dans un granulateur-mélangeur ; la granulation du mélange obtenu avec une solution ou dispersion de dexkétoprofène trométamol dans de l'eau; dans lequel la teneur en eau finale du granulat est d'environ 5 à environ 30 % en poids;

iv) la réalisation optionnelle et/ou si besoin d'un tamisage du granulat obtenu dans l'une ou l'autre de l'étape (ii) ou de l'étape (iii);

v) le séchage du granulat de l'étape (ii), (iii) ou (iv) précédente jusqu'à une teneur en eau finale inférieure à environ 8 % en poids, de préférence, inférieure à 6,5 % en poids, plus préférablement, d'environ 1,0 % à 5,5 % en poids, le séchage étant de préférence effectué dans un lit fluide à une température d'entrée comprise entre 35 et 105 °C.

13. Composition pharmaceutique orale à libération immédiate comprenant le granulat tel que défini dans la revendication 11 ou composition pharmaceutique orale selon la revendication 10 comprenant le granulat de la revendication 11.

14. Poudre comprenant le granulat tel que défini la revendication 11.

15. Utilisation d'un granulat selon la revendication 11 ou de la poudre selon la revendication 14 pour la fabrication d'une composition pharmaceutique orale, de préférence, d'un comprimé, de préférence encore, d'un comprimé enrobé.

Fig. 1

2Θ

Fig. 2

2Θ

Fig. 3

Fig. 4

Fig. 5

(min)

Fig. 6

(min)

Fig. 7

(min)

Fig. 8

(min)

**Fig. 9**

(min)

**Fig. 10**

(min)

**Fig. 11**

(min)

**Fig. 12**

(min)

**Fig. 13**

(min)

**Fig. 14**

(min)

**Fig. 15**

(min)

**Fig. 16**

(min)

**EP 2 671 569 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8904658 A
- WO 9411332 A
- EP 1739072 A
- US 5610712 A

**Non-patent literature cited in the description**

- **M.E. AULTON.** Pharmacuetics. The Science of Dosage Form Design. 2002, 404
- *Drug Development and Industrial Pharmacy,* 1994, vol. 20 (13), 2151-2156